# EUROPÄISCHE PATENTANMELDUNG

(11) **EP 1 938 789 A1**
(43) Veröffentlichungstag der Anmeldung: **02.07.2008**
(21) Anmeldenummer: 07023502.3
(22) Anmeldetag: 05.12.2007
(51) Int. Cl.: A61K 8/34, A61K 8/37, A61K 8/41, A61K 8/42, A61K 8/64, A61K 8/67, A61K 8/92, A61K 8/97, A61Q 19/06, A61Q 19/08

(54) **Verbesserung der Hautverträglichkeit von hyperämisierenden Wirkstoffen**

(30) Priorität: 19.12.2006 DE 102006060439
(71) Anmelder: Henkel AG & Co. KGaA, 40589 Düsseldorf (DE)
(72) Erfinder: Döring, Thomas, 41542 Dormagen (DE); Träger, Anemone, 40227 Düsseldorf (DE); Waldmann-Laue, Marianne, 40789 Monheim (DE); Anderheggen, Bernd, 42781 Haan (DE)

(57) **Zusammenfassung**

Die Erfindung betrifft topische kosmetische oder dermatologische Zusammensetzungen zur erwärmenden Behandlung der Haut, insbesondere zur Behandlung der Haut mit hyperämisierenden Wirkstoffen, mit verbesserter Hautverträglichkeit.

## Beschreibung

Die Erfindung betrifft topische kosmetische oder dermatologische Zusammensetzungen zur erwärmenden Behandlung der Haut, insbesondere zur Behandlung der Haut mit hyperämisierenden Wirkstoffen, mit verbesserter Hautverträglichkeit.

Bekannte Beispiele für hyperämisierende Wirkstoffe sind Terpentinöl, Nonivamid (auch als Pseudo-Capsaicin oder Nonansäurevanillylamid bezeichnet), Mauerpfeffer, Capsaicin, bestimmte Nicotinsäureester, insbesondere Nicotinsäurebenzylester (Benzylnicotinat), Nicotinsäurebutoxyethylester (auch als Nicoboxil bekannt), Nicotinsäuremethylester und Nicotinsäureethylester, aber auch die Hexyl-, Isopropyl- und Myristyl-Ester der Nicotinsäure, weiterhin Pyridin-3-carbaldehyd (β-Pyridincarbaldehyd, Nicotinaldehyd), bestimmte Salicylsäureester, insbesondere Phenylsalicylat, Vanillylbutylether sowie andere Vesikantien und Reiz-auslösende Stoffe. Unter Vesikantien (von lateinisch: vesica = Blase abgeleitete Bezeichnung) versteht man blasenziehende, das heißt, die Haut zur Erythem- und Blasenbildung mit Hyperämie reizende Mittel. Zu den Vesikantien zählen unter anderem Cantharidin, Capsaicin, Benzylsenföl und Präparate aus der Zaunrübe. Hyperämisierende Wirkstoffe werden häufig auch als Rubefacientien bezeichnet. Unter Rubefacientien (von lateinisch: rubefacere = röten abgeleitete Bezeichnung) versteht man allgemein Wirkstoffe, die infolge Hyperämie hautrötend beziehungsweise hautreizend wirken.

Derartige hyperämisierende Wirkstoffe werden in der Medizin als topische Anti-Rheuma-Mittel und Mittel gegen Muskelverspannungen eingesetzt. Bevorzugt werden der/die hyperämisierende Wirkstoff/e mit Hilfe von Salben, Gelen oder Pflastern aufgetragen.
In der Kosmetik werden hyperämisierende Wirkstoffe bevorzugt in der Cellulite-Behandlung eingesetzt.

Die hyperämisierende Wirkung wird von dem Verbraucher je nach allgemeinem Hautzustand, aber auch nach subjektivem Eindruck, als mehr oder weniger stark reizend und gegebenenfalls sogar als unangenehm empfunden. Diesem Nachteil abzuhelfen, war eine Aufgabe der vorliegenden Anmeldung.
Eine weitere Aufgabe der vorliegenden Anmeldung war es, die Hautverträglichkeit topischer Zusammensetzungen, die mindestens einen hyperämisierenden Wirkstoff enthalten, zu verbessern.

Eine weitere Aufgabe der vorliegenden Anmeldung war es, die Wirkung, insbesondere die Anti-Cellulite-Wirkung oder die Slimming-Wirkung topischer Zusammensetzungen, die mindestens einen hyperämisierenden Wirkstoff enthalten, zu verbessern.

Es hat sich überraschenderweise gezeigt, dass bei Einsatz von Vanillyl Butyl Ether und/oder Nicotinsäureestern (Methyl-, Ethyl-, Benzyl-, Butoxyethyl-, Hexyl-, Isopropyl-, Myristyl-, Tetrahydrofurfuryl-Nicotinat (Thurfyl-Nicotinat), Tetrahydrofurfuryl-Nicotinat-Hydrochlorid und/oder Capsaicin und/oder Capsaicin-haltiger Extrakte (z.B. Red Pepper Extract) und/oder Pseudocapsaicin (Hydroxymethoxybenzyl Pelargonamide) die Hautverträglichkeit entscheidend verbessert werden kann, wenn gleichzeitig Wirkstoffe eingesetzt werden, die in der Lage sind, die Degranulierung von Mastzellen und damit die Ausschüttung von Histamin zu reduzieren. Diese Wirkstoffkomposition bietet sich insbesondere für Körperpflegeprodukte wie z.B. ein Anti-Cellulite-Gel oder -Pflaster an. Überraschenderweise hat sich gezeigt, dass die Wirksamkeit von Anti-Cellulite-Produkten nachhaltig verbessert wird. Darüber hinaus sind sogar bei Anwendung im Gesichtsbereich gute Hautverträglichkeiten für *leave on-* und *rinse off*- Produkte möglich.

Überraschend wurde gefunden, dass Wirkstoffe, die in der Lage sind, die Degranulierung von Mastzellen und/oder die Ausschüttung von Histamin zu reduzieren, in Kombination mit hyperämisierenden Wirkstoffen in der Lage sind, deren Reizpotenzial verringern. Überraschend wurde weiterhin gefunden, dass Wirkstoffe, die in der Lage sind, die Degranulierung von Mastzellen und damit die Ausschüttung von Histamin zu reduzieren, in Kombination mit hyperämisierenden Wirkstoffen in der Lage sind, deren Reizpotenzial zu verringern, ohne deren Wirkung zu beeinträchtigen. Überraschend wurde weiterhin gefunden, dass Wirkstoffe, die in der Lage sind, die Degranulierung von Mastzellen und damit die Ausschüttung von Histamin zu reduzieren, in Kombination mit hyperämisierenden Wirkstoffen in der Lage sind, deren Reizpotenzial zu verringern und gleichzeitig deren Wirkung zu verbessern.

Mastzellen sind den basophilen Granulocyten ähnliche Zellen, die im Bindegewebe, in Blutgefäßwänden und im Lymphsystem vorkommen. Ihre basophilen Granula enthalten Histamin, Heparin, ATP, Proteinasen und andere entzündungsfördernde Mediatoren. Histamin ist in den Mastzellen in lockerem Komplex mit Heparin und Protein präformiert vorhanden. Es wird daraus auf einen entsprechenden Stimulus hin schlagartig freigesetzt. Auslöser können mechanische Verletzungen, Traumata, allergische Reaktionen, Infektionen und Einwirkung von Toxinen sein. Vermehrt tritt Histamin bei Allergien und Anaphylaxien auf, wobei es aufgrund der Bindung des Allergens an Membran-gebundenes Immunglobulin E aus Mastzellen ausgeschüttet wird. Dabei löst die Antigen-Antikörper-Reaktion eines Allergens mit an der Mastzellen-Oberfläche gebundenen IgE (Immunglobulinen E) eine Abgabe des Inhalts der Granula nach außen, die sogenannte Degranulierung, aus.

Gegenstand der vorliegenden Erfindung sind kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten.

Erfindungsgemäß bevorzugte kosmetische oder dermatologische Zusammensetzungen sind dadurch gekennzeichnet, dass sie mindestens einen hyperämisierenden Wirkstoff enthalten, der ausgewählt ist aus
- Nicotinsäureestern, insbesondere Nicotinsäurebenzylester (Benzylnicotinat), Nicotinsäurebutoxyethylester (Nicoboxil), Nicotinsäuremethylester, Nicotinsäureethylester, Nicotinsäurehexylester, Nicotinsäureisopropylester, Nicotinsäuremyristylester, Tetrahydrofurfuryl-Nicotinat (Thurfyl-Nicotinat) und Tetrahydrofurfuryl-Nicotinat-Hydrochlorid,
- Pyridin-3-carbaldehyd (β-Pyridincarbaldehyd, Nicotinaldehyd),
- Salicylsäureestern, insbesondere Phenylsalicylat,
- Vanillylethern, insbesondere Vanillylbutylether,
- Carbonsäurevanillylamiden, insbesondere Nonivamid (Nonansäurevanillylamid),
- Scharfstoffen, insbesondere Capsaicin, Cantharidin, Piperin, Gingerol, Zingeron, Myristicin, Safrol, Allicin, Sedamin, Sacculatal, Chalciporon, Isochalciporon, Velleral, Vellerol, und Isothiocyanaten (Senfölen), insbesondere Benzylsenföl,
- Extrakten aus Scharfstoff-Pflanzen, insbesondere aus Capsicum, insbesondere aus Capsicum annuum und Capsicum frutescens, aber auch aus Capsicum chinense, Capsicum baccatum und Capsicum pubescens, weiterhin aus Mauerpfeffer und aus der Zaunrübe,
- Terpentinöl,
- sowie Mischungen hiervon.

Nachfolgend sind die erfindungsgemäß bevorzugten hyperämisierenden Wirkstoffe näher erläutert.

### Nonivamid

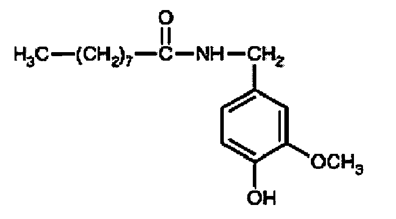

Nonivamid = Internationaler Freiname für N-(4-Hydroxy-3-methoxybenzyl)nonanamid (Pelargonsäure- oder Nonansäurevanillylamid, Pseudocapsaicin), C₁₇H₂₇NO₃, *M*ᵣ 293,40, Schmelzpunkt 52-55°C. Nonivamid ist als hyperämisierender Wirkstoff in Pflastern und Salben als Generikum im Handel.

### Vanillylbutylether

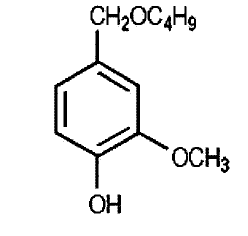

("Vanillyl..." ist erfindungsgemäß die veraltete Bezeichnung (in Anlehnung an die überholte IUPAC-Regel C-411.1) für die systematisch "(4-Hydroxy-3-methoxybenzyl)..." oder gemäß Chemical Abstracts: [(4-Hydroxy-3-methoxyphenyl)methyl]... genannte Atomgruppierung.)

Bevorzugte Scharfstoffe bzw. Scharfstoff-Pflanzen sind: Capsaicin aus Paprika, Piperin aus Pfeffer, Gingerol und Zingeron aus Ingwer, Myristicin aus Muskat, Allicin aus Knoblauch, Sedamin aus Mauerpfeffer (Sedum-Alkaloid), Sacculatal aus Lebermoosen, Chalciporon aus dem PfefferRöhrling *(Suillus piperatus),* Velleral aus Milchlings-(Lactarius)-Arten, Isothiocyanate (Senföle) aus Senf, Meerrettich, Kresse und anderen Kreuzblütlern (Brassicaceae).

Capsaicin [(*E*)-*N*-(4-Hydroxy-3-methoxybenzyl)-8-methyl-6-nonenamid; FEMA 3404].

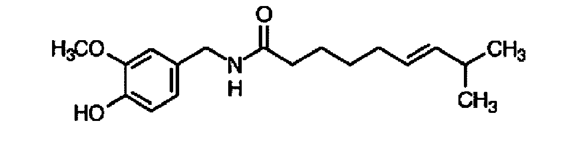

C₁₈H₂₇NO₃, *M*ᵣ 305,42. Monokline Kristalle, Schmelzpunkt 64-65°C, in Wasser kaum, in den meisten organischen Lösemitteln gut löslich. Capsaicin verursacht den scharfen Geschmack der Paprika-Früchte, Chillies und anderen Capsicum-Arten (noch in einer Verdünnung von 1:10⁵), in denen es zu 0,3 - 0,5 Gew.-% enthalten ist.

Piperin (1-Piperoylpiperidin).

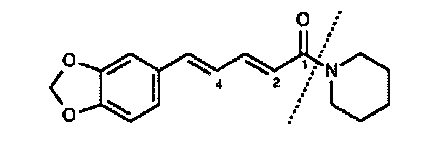

C₁₇H₁₉NO₃, *M*ᵣ 285,34, Schmelzpunkt 128-129°C. Piperin findet sich zu 5-9% im Pfeffer *(Piper nigrum)* als dessen wichtigster Scharfstoff und in vielen weiteren *Piper*-Arten (Piperaceae). Neben Piperin sind über 50 analoge Amide, meist mit Piperidin, Pyrrolidin Schmelzpunkt Isobutylamin als Basenteil aus *Piper*-Arten bekannt.

Gingerol [6-Gingerol, 5-Hydroxy-1-(4-hydroxy-3-methoxyphenyl)-3-decanon].

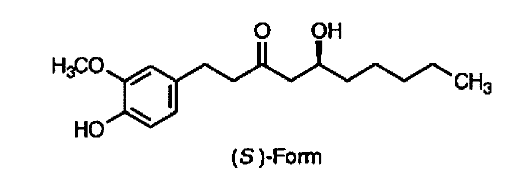

C₁₇H₂₆O₄, *M*ᵣ 294,39, (S)-Form, gelbes Öl, löslich in organischen Lösemitteln, Scharfstoff in Ingwer *(Zingiber officinale).* (R)-Form, gelbes Öl. Das Racemat besitzt ähnliche Geschmackseigenschaften wie das (S)-Enantiomer, es fehlt jedoch der typische Ingwergeruch.

### Zingeron [Zingiberon, Vanillylaceton, 4-(4-Hydroxy-3-methoxyphenyl)butan-2-on; FEMA 3124]

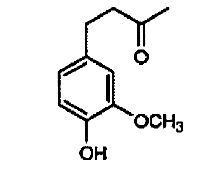

C₁₁H₁₄O₃, *M*ᵣ 194,22, farblose, scharf schmeckende Kristalle, Schmelzpunkt 40°C, wenig löslich in Wasser, löslich in Ether und verdünnten Alkalien. Zingeron ist der Scharfstoff im Oleoresin des Ingwers, aus dem es isoliert werden kann.

### Myristicin, Safrol

[5-(2-Propenyl)-1,3-benzodioxol, 4-Allyl-1,2-methylendioxybenzol].

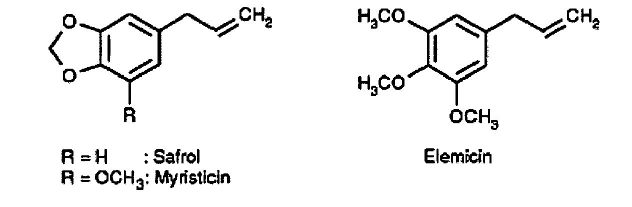

C₁₀H₁₀O₂, *M*ᵣ 162,19, farblose bis blassgelbe Flüssigkeit, Geruch nach Safran, Siedepunkt 232-234°C, Prismen, Schmelzpunkt 11°C. Safrol ist Hauptbestandteil des Sassafrasöls (bis zu 90%) und kommt in größeren Mengen auch in Campheröl vor. In zahlreichen anderen essentiellen Ölen, unter anderem in Sternanis, Basilikum, Fenchel, Anis, Zimt, schwarzem Pfeffer und in Muskatnüssen ist Safrol neben kleineren Mengen Myristicin enthalten.

*Myristicin* [4-Methoxy-6-(2-propenyl)-1,3-benzodioxol], C₁₁H₁₂O₃, *M*ᵣ 192,21, Öl, Siedepunkt 149-149,5°C (2 kPa), 95-97°C (27 Pa), löslich in Ether und Benzol, kommt unter anderem im Petersilienöl, in Möhren, in Anisöl und in der Muskatnuss vor, wo es von Safrol und *Elemicin* [1,2,3-Trimethoxy-5-(2-propenyl)benzol] begleitet ist. Myristicin verursacht den typischen Muskatnussduft.

### Allium

Die Biosynthese der Schwefel-haltigen Inhaltsstoffe führt vom Sulfat über Cystein zu Glutathion. Die SH-Gruppen dieser beiden Peptide werden mit Methacrylsäure (aus Valin) alkyliert bzw. bei Gluthathion auch methyliert.

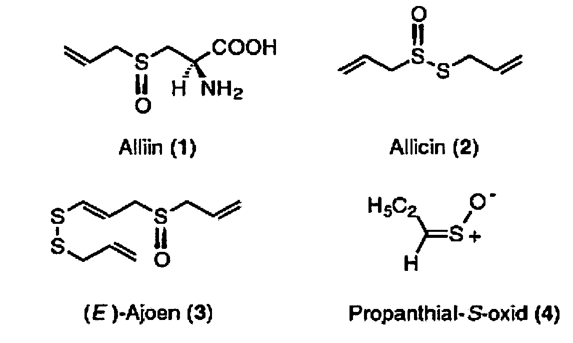

### Mauerpfeffer, Sedacin

Am C-2 monosubstituierte Piperidin-Alkaloide aus Sedum-Arten (Crassulaceae). Für den scharfen Geschmack des scharfen Mauerpfeffers *(Sedum acre*) ist *Sedamin* verantwortlich.

### Sedamin

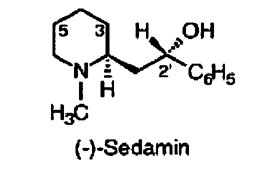

C₁₄H₂₁NO, *M*ᵣ 219,33, Schmelzpunkt 75-76°C. Mit Sedamin verwandt sind u.a. 5-Hydroxysedamin, das 2'-Epimer Allosedamin, dessen N-Demethyl-Derivat Norallosedamin und die beiden dazugehörigen Hydroxy-Derivate 3-Hydroxyallosedamin und 3-Hydroxynorallosedamin.

### Sacculatal

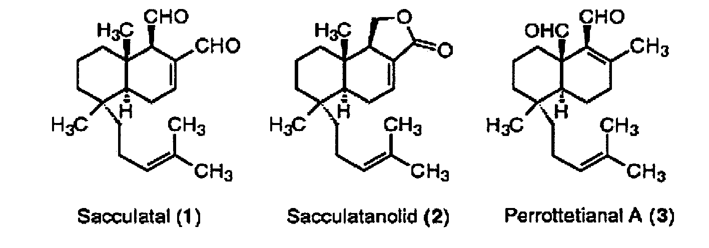

Diterpene wie *Sacculatal, Sacculatanolid* und *Perrottetianal A* (alle: C₂₀H₃₀O₂, *M*ᵣ 302,46), die das bislang nur bei Lebermoosen anzutreffende Sacculatan-Grundgerüst aufweisen. Sacculatal, Schmelzpunkt 65 -66 °C, ist für den stechend scharfen Geschmack der Thalli von *Pellia endiviifolia* und *Trichocoleopsis sacculata* mitverantwortlich.

### Chalciporon

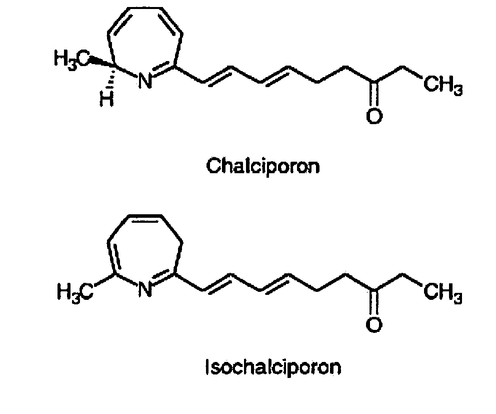

C₁₆H₂₁NO, *M*ᵣ 243,35, gelbliches Öl. Alkaloid aus dem Pfefferröhrling *(Chalciporus piperatus,* Basidiomycetes), das für den brennenden Geschmack des Pilzes verantwortlich ist. Neben Chalciporon und einigen verwandten 2H-Azepinen wurden auch die entsprechenden 3H-Azepine (z.B. *Isochalciporon,* hellgelbes Öl) isoliert, die aus den 2*H*-Isomeren durch sigmatrope [1,5]-H-Verschiebung entstehen, achiral sind und mild schmecken.

### Velleral

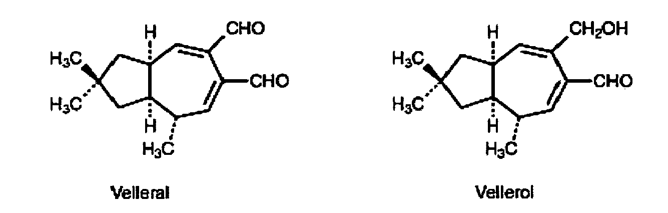

C₁₅H₂₀O₂, *M*ᵣ 232,32, Kristalle, Schmelzpunkt 86,5-87,5°C. Sesquiterpen aus Pilzen der Gattung *Lactarius* (Milchlinge), *Russula* (Täublinge) und anderen Russulaceae.

### Senföle

Historisch bedingte Bezeichnung für organische Isothiocyanate, R-N=C=S, besonders für solche, die als stechend riechende, geschmacksgebende Bestandteile der etherischen Öle vorwiegend in Brassicaceae (Kreuzblütlern) vorkommen. Die Senföle liegen dort glycosidisch gebunden als Glucosinolate vor, aus denen sie - unter Umlagerung - durch Thioglucosidasen (Beispiel: Myrosinase) freigesetzt werden.
Verbreitete Senföle sind Allylsenföl (Allylisothiocyanat), 3-Methylthiopropylsenföl, Butylsenföl, 3-Butenylsenföl, Erucin, Erysolin, Hirsutin, Isopropylsenföl, β-Phenylethylsenföl, Sulforaphan und Sulforaphen (Raphanin).

Entsprechende hyperämisierende Wirkstoffe sind im Handel erhältlich, teilweise in für kosmetische oder dermatologische Zusammensetzungen aufbereiteter Form. Erfindungsgemäß außerordentlich bevorzugte Wirkstoffe sind Ethylnicotinat, Vanillylbutylether, z.B. der Rohstoff Hotact VBE von Takasago, Capsaicin, erhältlich z. B. als Rohstoff Herbasol Extract Capsicum (INCI: Propylene Glycol, Aqua (Water), Capsicum Frutescens Fruit Extract, Sorbitol) und N-Vanillylnonamid (Pseudocapsaicin).
Bevorzugte erfindungsgemäße Zusammensetzungen sind dadurch gekennzeichnet, dass sie mindestens einen hyperämisierenden Wirkstoff in einer Aktivsubstanz-Gesamtmenge von 0,005 - 3,0 Gew.-%, bevorzugt 0,01 - 1,0, besonders bevorzugt 0,05 - 0,5 Gew.-% und außerordentlich bevorzugt 0,1 - 0,35 Gew.%, jeweils bezogen auf das Gesamtgewicht der erfindungsgemäßen Zusammensetzung, enthalten.

Weitere erfindungsgemäß bevorzugte kosmetische oder dermatologische Zusammensetzungen sind dadurch gekennzeichnet, dass sie mindestens einen Wirkstoff, der die Degranulierung von Mastzellen und/oder die Ausschüttung von Histamin reduziert, enthalten, der ausgewählt ist aus
- Extrakten aus den Samen von Cucurbita pepo (Zucchini),
- Extrakten aus Mentha piperita, insbesondere aus den Blättern von Mentha piperita,
- N-acylierten Alkanolaminen, insbesondere N-Palmitoylethanolamin,
- Hydrolysaten oder Extrakten aus der Alge *Enteromorpha compressa* (Darmtang, eine Grünalge), insbesondere aus dem Vegetationskörper (Thallus) der Alge, z. B. in Enteline 2,
- dem Dipeptid Tyr-Arg, dessen Estern und dessen N-Acyl-Derivaten, insbesondere N-Acetyl-Tyr-Arg-hexyldecylester (z. B. Calmosensine von Sederma),
- sowie Mischungen hiervon.

Es kann erfindungsgemäß bevorzugt sein, dass die die Degranulierung von Mastzellen und/oder die Ausschüttung von Histamin reduzierenden Wirkstoffe b), die als Extrakt oder Hydrolysat einer Pflanze, Alge oder ähnlicher Organismen oder Teilen dieser Organismen erhältlich sind, in wasserlöslicher Form vorliegen.
Erfindungsgemäß wird unter Wasserlöslichkeit eine Löslichkeit von wenigstens 5 Gew.-% bei 20 °C verstanden, das heißt, dass Mengen von wenigstens 5 g des Wirkstoffes in 95 g Wasser bei 20 °C löslich sind.

Erfindungsgemäß besonders bevorzugte Extrakte aus den Samen von Cucurbita pepo (Zucchini) sind beispielsweise in den Handelsprodukten Curbilene (ex Indena SpA, INCI: Cucurbita pepo (pumpkin) seed extract), Ocaline (ex Soliance, INCI: Sea Water (and) Water (and) Cucurbita pepo (pumpkin) seed extract), ARP 100 (ex Greentech, INCI: Water, Alcohol, Serenoa Serrulata Fruit Extract, Epilobium Angustifolium Extract, Cucurbita Pepo (Pumpkin) Seed Extract), ARP 100 Huileux (ex Greentech, INCI: Caprylic/Capric Triglyceride, Serenoa Serrulata Fruit Extract, Epilobium Angustifolium Flower/Leaf/Stem Extract, Cucurbita Pepo (Pumpkin) Seed Extract), Cucurbitine (ex Christian Dior Parfums, INCI: Water (and) Cucurbita pepo (pumpkin) seed extract), Herbasol Extract Pumpkin (ex Cosmetochem, INCI: Water (and) Propylene glycol (and) Cucurbita pepo (pumpkin) seed extract) und Pumpkin Extract (ex Draco Natural Products, INCI: Water (and) Cucurbita pepo (pumpkin) seed extract) enthalten.
Erfindungsgemäß besonders bevorzugte Extrakte aus Mentha piperita, insbesondere aus den Blättern von Mentha piperita, sind beispielsweise in den Handelsprodukten Calmiskin OP (ex Silab, INCI: Water (and) Mentha piperita (Peppermint) Leaf Extract) und Caomint (ex Solabia, INCI: Propylene Glycol, Water, Mentha piperita (Peppermint) Leaf Extract, Theobroma Cacao (Cocoa) Extract) enthalten.

Weitere erfindungsgemäß bevorzugte Wirkstoffe, die die Degranulierung von Mastzellen und/oder die Ausschüttung von Histamin reduzieren können, sind ausgewählt aus N-acylierten Alkanolaminen. Erfindungsgemäß bevorzugt sind die Alkanolamine von Methanol, Ethanol, 1-Propanol, 2-Propanol, Isopropanol, 1-Butanol, 2-Butanol, sek.-Butanol, tert.-Butanol und höheren Alkanolen, insbesondere den Isomeren von Pentanol, Hexanol, Heptanol, Octanol, Nonanol und Decanol.
Entsprechende erfindungsgemäße Wirkstoffe können Monoalkonolamine und Dialkanolamine sein. Aufgrund der Gefahr einer Bildung von Nitrosaminen ist es jedoch außerordentlich bevorzugt, dass die Wirkstoffe möglichst frei von Dialkanolaminen sind; die Monoalkanolamine sind daher außerordentlich bevorzugt.
Besonders bevorzugt ist N-acyliertes Monoethanolamin. Zur N-Acylierung besonders bevorzugt sind C₈-C₁₈-Fettsäuren, insbesondere Octansäure, Nonansäure, Decansäure, Undecansäure, Dodecansäure, Myristinsäure (C₁₄), Palmitinsäure (C₁₆) und Stearinsäure, wobei Myristinsäure und Palmitinsäure ganz besonders bevorzugt sind. Ein erfindungsgemäß außerordentlich bevorzugtes N-acyliertes Monoalkanolamin ist N-Palmitoylethanolamin.

Weitere erfindungsgemäß bevorzugte Wirkstoffe, die die Degranulierung von Mastzellen und/oder die Ausschüttung von Histamin reduzieren, sind ausgewählt aus Hydrolysaten oder Extrakten aus der Grünalge *Enteromorpha compressa* (Darmtang). Ein erfindungsgemäß besonders bevorzugtes Hydrolysat aus *Enteromorpha compressa,* insbesondere aus dem Vegetationskörper (Thallus) von *Enteromorpha compressa,* ist beispielsweise in dem Handelsprodukt Enteline 2 (INCI: Butylene Glycol, Glycerine, Hydrolyzed Enteromorpha compressa) von BiotechMarine enthalten.

Weitere erfindungsgemäß bevorzugte Wirkstoffe, die die Degranulierung von Mastzellen und/oder die Ausschüttung von Histamin reduzieren, sind ausgewählt aus dem Dipeptid Tyr-Arg, dessen Estern und dessen N-Acyl-Derivaten, insbesondere Verbindungen wie N-Acetyl-Tyr-Arg-hexyldecylester, das beispielsweise in dem Rohstoff Calmosensine (INCI: Butylene Glycol, Aqua (Water), Laureth-3, Hydroxyethylcellulose, Acetyl Dipeptide-1 Cetyl Ester) von Sederma enthalten ist.

Bevorzugte erfindungsgemäße Zusammensetzungen sind dadurch gekennzeichnet, dass sie mindestens einen Wirkstoff, der die Degranulierung von Mastzellen und/oder die Ausschüttung von Histamin reduziert, in einer Aktivsubstanz-Gesamtmenge von 0,001 - 2,0 Gew.-%, bevorzugt 0,01 - 1,0, besonders bevorzugt 0,05 - 0,5 und außerordentlich bevorzugt 0,1 - 0,2 Gew.%, jeweils bezogen auf das Gesamtgewicht der erfindungsgemäßen Zusammensetzung, enthalten.

Es kann erfindungsgemäß bevorzugt sein, dass die als Wirkstoff a) oder b) eingesetzten Aminosäuren und Peptide zur Verbesserung der Penetration in die Haut mit mindestens einer, bevorzugt linearen, C₂-C₂₂-Fettsäure N-acyliert und/oder verestert sind. Zur N-Acylierung und/oder Veresterung besonders bevorzugt sind C₈-C₁₈-Fettsäuren, ganz besonders bevorzugt sind Myristinsäure (C₁₄) und Palmitinsäure (C₁₆). Besonders bevorzugt ist weiterhin, dass alle verwendeten Aminosäuren und Peptide derartig N-acyliert und/oder verestert sind. Ebenfalls bevorzugt ist die Substitution der Aminosäuren und Peptide mit einer Benzyloxycarbonylgruppe an der terminalen Aminogruppe.

Die Wirkung der erfindungsgemäßen Wirkstoffkombination, insbesondere in Bezug auf die Verbesserung der Oberflächenstruktur der Haut, kann weiter gesteigert werden durch mindestens einen Wirkstoff, der die Kollagensynthese stimuliert. Ohne an diese Theorie gebunden sein zu wollen, wird vermutet, dass die durchblutungsfördernde Wirkung des hyperämisierenden Wirkstoffs auch die Wirksamkeit des die Kollagensynthese stimulierenden Wirkstoffs begünstigt. Dies ist allerdings insoweit überraschend, da die Anticellulite-Wirkung andere Hautschichten betrifft als die Kollagensynthese. Weitere erfindungsgemäß bevorzugte Zusammensetzungen sind daher dadurch gekennzeichnet, dasss mindestens ein Wirkstoff enthalten ist, der die Kollagensynthese stimuliert. Erfindungsgemäß besonders bevorzugte die Kollagensynthese stimulierende Wirkstoffe sind ausgewählt aus Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist, weiterhin aus den Tripeptiden Gly-His-Lys, Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure) und Dap-Val-Lys, weiterhin aus dem Tetrapeptid Rigin, Rigin-basierten Tetrapeptiden sowie ALAMCAT-Tetrapeptiden, dem Pentapeptid Lys-Thr-Thr-Lys-Ser, den Hexapeptiden Val-Gly-Val-Ala-Pro-Gly, Ala-Asp-Leu-Lys-Pro-Thr (Hexapeptide-3), Hexapeptide-4, Hexapeptide-5, Hexapeptide-6, Hexapeptide-8, Hexapeptide-9 und Hexapeptide-10, weiterhin ausgewählt aus Sojaproteinhydrolysaten mit einer mittleren Molmasse im Bereich von 600 - 1000 Dalton, bevorzugt 800 Dalton, wobei die genannten Peptide in N-acylierter Form vorliegen können, was erfindungsgemäß besonders bevorzugt sein kann, weiterhin ausgewählt aus Retinoiden, Kombucha, Olivenblattextrakten, Oleanolsäure, Oleanol, Extrakten aus Haferkörnern (Avena Sativa (Oat) Kernel Extract) und liposomenverkapselten Grüntee-Extrakten.
Das Tripeptid Gly-His-Lys ist z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich, stellt aber (in acylierter Form) auch einen Bestandteil des Produktes Matrixyl 3000 von Sederma dar. Das Tripeptid Gly-His-Lys kann auch als Kupfersalz (Cu²⁺) eingesetzt werden und ist als solches über ProCyte Corporation zu beziehen. Weiterhin können bevorzugt Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile bevorzugt geeignet. Die erfindungsgemäß besonders bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn oder Citrullin ersetzt. Ein weiteres erfindungsgemäß bevorzugtes Peptid, das die Kollagenynthese stimuliert, ist dementsprechend Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist.
Weitere erfindungsgemäß bevorzugte Tripeptide, die die Kollagenynthese stimulieren, sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure) und Dap-Val-Lys. Ein besonders bevorzugtes Tripeptid ist Palmitoyl-Lys-Val-Lys, z. B. erhältlich von der Firma Pentapharm unter der Bezeichnung SYN^{®}-COLL.

Weitere erfindungsgemäß bevorzugte Tetrapeptide, die die Kollagenynthese stimulieren, sind das Tetrapeptid Rigin und Rigin-basierte Tetrapeptide sowie ALAMCAT-Tetrapeptide. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg (INCI-Bezeichnung: Palmitoyl Tetrapeptide-1), das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapeptide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gin und den Aminosäuren, die Gln substituieren können, Xcc ausgewählt ist aus Pro oder Arg und den Aminosäuren, die Pro und Arg substituieren können. Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (lle).
Die bevorzugten Aminosäuren, die Gin ersetzen können, beinhalten eine Seitenkette mit einer Aminogruppe, die bei neutralem pH (pH 6-7) überwiegend ungeladen vorliegt, z. B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.

Weitere erfindungsgemäß bevorzugte Peptide, die die Kollagensynthese stimulieren, sind das Pentapeptid Lys-Thr-Thr-Lys-Ser und seine N-acylierten Derivate, besonders bevorzugt N-Palmitoyl-Lys-Thr-Thr-Lys-Ser, das unter der Bezeichnung Matrixyl von der Firma Sederma erhältlich ist.

Weitere erfindungsgemäß bevorzugte Hexapeptide, die die Kollagensynthese stimulieren, sind das Hexapeptid Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist. Weitere erfindungsgemäß bevorzugte Peptide sind die Hexapeptide und/oder deren N-acylierten Derivate Ala-Asp-Leu-Lys-Pro-Thr (Hexapeptide-3, z. B. Peptide 02 von Vincience), Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec).

Weitere erfindungsgemäß bevorzugte Wirkstoffe, die die Kollagensynthese stimulieren, sind ausgewählt aus Sojaproteinhydrolysaten mit einer mittleren Molmasse im Bereich von 600 - 1000 Dalton, bevorzugt 800 Dalton. Ein derartiges besonders bevorzugtes Sojaproteinhydrolysat ist unter dem Handelsnamen Phytokine von der Firma Coletica erhältlich.

Weitere erfindungsgemäß bevorzugte Wirkstoffe, die die Kollagensynthese stimulieren, sind ausgewählt aus Retinoiden. Zu den Retinoiden werden im Sinne der vorliegenden Erfindung Retinol (Vitamin A₁), die C₂-C₂₂-Fettsäureester des Retinols (= Retinylester, insbesondere Retinylpalmitat und Retinylacetat), 3,4-Didehydroretinol (Vitamin A₂), Retinal und seine Isomere, *all-trans*-Retinsäure, 9-cis-Retinsäure und 13-cis-Retinsäure (Tretinoin), die Ester der Retinsäure sowie weitere verwandte Substanzen (synthetische Retinoide) gezählt, die für ihre vielfältige biologische Wirkung insbesondere auf Wachstum und Differenzierung bekannt sind, sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen. Synthetische Retinoide werden in drei Gruppen eingeteilt: nichtaromatisch (z. B. *Isotretinoin),* monoaromatisch (z.B. Acitretin) und polyaromatisch (sogenannte Arotinoide, z. B. Tazarotene, die eine spezifische Wirkung auf einzelne Retinoid-Rezeptoren haben). Erfindungsgemäß besonders bevorzugte Retinoide sind Retinol und die C₂-C₂₂-Fettsäureester des Retinols, insbesondere Retinylpalmitat und Retinylacetat.

Weitere erfindungsgemäß bevorzugte Wirkstoffe, die die Kollagensynthese stimulieren, sind ausgewählt aus Kombucha.
Bei Kombucha handelt es sich um ein aus dem ostasiatischen Raum stammendes, etwa 0,5 Vol.-% Alkohol enthaltendes, angenehm säuerliches, leicht süßes, moussierendes Gärungsgetränk auf Basis von Mono- oder Disaccharid-haltigem Tee (Schwarzer Tee, Grüner Tee, Kräutertee oder Früchtetee), das ohne Zusatz von Konservierungsstoffen, Farbstoffen und Aromastoffen hergestellt wird.
Beim "Kombucha-Teepilz" handelt es sich tatsächlich nicht um einen Pilz, sondern um eine Symbiose von Essigsäurebakterien (meistens *Acetobacterxylinum,* aber auch *Acetobactergluconicum)* mit verschiedenen Hefen *(Saccharomyces* sp., *Torula* sp., *Pichia fermentans);* fakultativ können Milchsäurebakterien vorhanden sein. Aus Gründen der Produktionssicherheit und Produktionsstabilität wird industriell gefertigter Kombucha oft wärmebehandelt.

Unter "Tee" werden erfindungsgemäß wässrige Aufgüsse von Blättern, Knospen und zarten Stielen von Thea sinensis (Camellia sinensis) und Thea assamica, aber auch von diversen anderen Pflanzen verstanden, beispielsweise Linde (Lindenblüten), Malve, Rotbusch, Kamille usw.

Bevorzugte Kombucha ist aus Tee von fermentierten (Schwarztee, Oolong-Tee, Gelber Tee, Pu-Erh-Tee) oder unfermentierten (Grüntee) Blättern von Thea sinensis (Camellia sinensis) oder Thea assamica gewonnen. Weitere bevorzugte Kombucha ist durch Fermentation von (meist mit Saccharose) gezuckertem Tee mit den symbiotischen Mikroorganismen Saccharomyces und Acetobacter xylinum gewonnen. Ein erfindungsgemäß besonders bevorzugtes Kombucha-Produkt ist ein Produkt, das durch Fermentation von mit Saccharose gezuckertem Schwarztee mit den zwei symbiotischen Mikroorganismen Saccharomyces und Acetobacter xylinum gewonnen wurde und beispielsweise unter der INCI-Bezeichnung "Saccharomyces/Xylinum/Black Tea Ferment, Glycerin, Hydroxyethylcellulose" mit der Handelsbezeichnung "Kombuchka" als Glycerin-haltige verdickte Zubereitung von der Firma Sederma erhältlich ist.

Weitere erfindungsgemäß bevorzugte Wirkstoffe, die die Kollagensynthese stimulieren, sind ausgewählt aus Olivenblattextrakten (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.

Olivenblattextrakte können einen hohen Gehalt an Oleanolsäure und/oder Oleanol aufweisen. Weitere erfindungsgemäß bevorzugte Wirkstoffe, die die Kollagensynthese stimulieren, sind ausgewählt aus Oleanolsäure und Oleanol.

Weitere erfindungsgemäß bevorzugte Wirkstoffe, die die Kollagensynthese stimulieren, sind ausgewählt aus Extrakten aus Haferkörnern (Avena Sativa (Oat) Kernel Extract). Erfindungsgemäß besonders bevorzugte Extrakte aus Haferkörnern sind durch einen Gehalt an beta-Glucan gekennzeichnet, dass zu mindestens 60 %, bevorzugt 70 % beta-1,4-glucosidische Bindungen aufweist. Ein erfindungsgemäß besonders bevorzugter Extrakt aus Haferkörnern ist unter der Handelsbezeichnung Drago Beta Glucan (02/060800) von der Firma Symrise erhältlich.

Weitere erfindungsgemäß bevorzugte Wirkstoffe, die die Kollagensynthese stimulieren, sind ausgewählt aus liposomenverkapselten Grüntee-Extrakten (Camellia Sinensis). Ein erfindungsgemäß besonders bevorzugter liposomenverkapselter Grüntee-Extrakt ist unter der Handelsbezeichnung Greenselect Phytosome von der Firma Indena erhältlich.

Erfindungsgemäß besonders bevorzugte kosmetische oder dermatologische Zusammensetzungen sind dadurch gekennzeichnet, dass sie neben der erfindungsgemäßen Wirkstoffkombination mindestens einen Wirkstoff, der die Kollagensynthese stimuliert, in einer Gesamtmenge von 0,000001 - 5 Gew.-%, bevorzugt 0,00001 - 2 Gew.-%, besonders bevorzugt 0,0001 - 1 Gew.-% und außerordentlich bevorzugt 0,005 - 0,5 Gew.%, enthalten, jeweils bezogen auf den Gehalt an Aktivsubstanz in der gesamten Zusammensetzung.

Die Wirkung der erfindungsgemäßen Wirkstoffkombination, insbesondere in Bezug auf die Verbesserung der Elastizität und/oder des Feuchtigkeitsgehaltes der Haut, kann weiter gesteigert werden durch mindestens einen Wirkstoff, der die Interaktion zwischen der extrazellulären Matrix und den Fibroblasten erhöht und/oder verbessert. Ohne an diese Theorie gebunden sein zu wollen, wird vermutet, dass die durchblutungsfördernde Wirkung des hyperämisierenden Wirkstoffs auch die Wirksamkeit des die Interaktion zwischen der extrazellulären Matrix und den Fibroblasten erhöhenden und/oder verbessernden Wirkstoffs begünstigt. Dies ist allerdings insoweit überraschend, da die Anticellulite-Wirkung andere Hautschichten betrifft. Weitere erfindungsgemäß bevorzugte Zusammensetzungen sind daher dadurch gekennzeichnet, dasss mindestens ein Wirkstoff enthalten ist, der die Interaktion zwischen der extrazellulären Matrix und den Fibroblasten erhöht und/oder verbessert. Weitere erfindungsgemäß bevorzugte kosmetische oder dermatologische Zusammensetzungen sind dadurch gekennzeichnet, dass sie mindestens einen Wirkstoff enthalten, der die Interaktion zwischen der extrazellulären Matrix und den Fibroblasten erhöht und/oder verbessert und der ausgewählt ist aus
- Sojaproteinhydrolysaten mit einer mittleren Molmasse im Bereich von 1200 - 1800 Dalton, bevorzugt im Bereich von 1400 - 1700 Dalton,
- mit Kokosfettsäuren N-acylierten und/oder veresterten Sojaproteinhydrolysaten in Form ihrer Alkalimetallsalze, bevorzugt in Form der Kaliumsalze,
- Ascorbinsäure, den Estern der Ascorbinsäure mit anorganischen und/oder organischen Säuren, den Ethern der Ascorbinsäure mit Mono-, Oligo- und Polysacchariden sowie den physiologisch verträglichen Salzen dieser Komponenten,
- Apfelkernextrakten (Pyrus Malus (Apple) Fruit Extract),
- Lotuskeim-Extrakten (Nelumbo Nucifera Germ Extract),
- Extrakten aus Maiskörnern (Zea Mays (Corn) Kernel Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Hydroxystilbenen und deren Estern, insbesondere Resveratrol und/oder Resveratrolmono-,
- di- und -triphosphorsäureestern und deren Salzen,
- Keratinhydrolysaten, insbesondere Wollkeratinhydrolysaten,
- Conchiolinhydrolysaten,
- dem Dipeptid L-Citrullyl-L-arginin und seinen mit mindestens einer, bevorzugt linearen, C₂-C₂₂-Fettsäure N-acylierten und/oder veresterten Derivaten, bevorzugt Acetyl Citrull Amido Arginine,
- Carnitin,
- Hypotaurin,
- L-Glutamylaminoethyl-indol,
- Isoflavonoiden und Isoflavonoid-reichen Pflanzenextrakten,
- Dihydroquercetin (= Taxifolin),
- sowie Mischungen hiervon.

Erfindungsgemäß bevorzugte Wirkstoffe, die die Interaktion zwischen der extrazellulären Matrix und den Fibroblasten erhöhen und/oder verbessern, sind ausgewählt aus Sojaproteinhydrolysaten mit einer mittleren Molmasse (Mw) im Bereich von 1200 - 1800 Dalton, bevorzugt im Bereich von 1400 - 1700 Dalton (Da). Ein derartiges besonders bevorzugtes Sojaproteinhydrolysat ist unter dem Handelsnamen Ridulisse C von der Firma Silab erhältlich. Ridulisse C enthält vier Molmassenfraktionen: 0,8 Gew.-% mit einem Mw > 12.500 Da, 22,2 Gew.-% mit 1355 Da < Mw < 12.500 Da, 43,1 Gew.-% 75 Da < Mw < 1355 Da und 33,9 Gew.-% < 75 Da.

Weitere erfindungsgemäß bevorzugte Wirkstoffe, die die Interaktion zwischen der extrazellulären Matrix und den Fibroblasten erhöhen und/oder verbessern, sind ausgewählt aus mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 -18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.
Ein erfindungsgemäß besonders bevorzugtes, mit Kokosfettsäuren N-acyliertes und/oder verestertes Sojaproteinhydrolysat in Form des Kaliumsalzes ist unter der Handelsbezeichnung Coccopolipeptide di Soja von der Firma Sinerga erhältlich

Weitere erfindungsgemäß bevorzugte Wirkstoffe, die die Interaktion zwischen der extrazellulären Matrix und den Fibroblasten erhöhen und/oder verbessern, sind ausgewählt aus Ascorbinsäure, den Estern der Ascorbinsäure mit anorganischen und/oder organischen Säuren, den Ethern der Ascorbinsäure mit Mono-, Oligo- und Polysacchariden, sowie den physiologisch verträglichen Salze dieser Komponenten. Bei den physiologisch verträglichen Salzen sind insbesondere die Zink-, Kupfer- und Mangansalze und die Salze der Alkali- und der Erdalkalimetalle bevorzugt, besonders die Natrium-, Kalium-, Magnesium- und Calcium-Salze. Erfindungsgemäß besonders bevorzugte kosmetische oder dermatologische Zusammensetzungen sind dadurch gekennzeichnet, dass sie mindestens einen Wirkstoff enthalten, der die Interaktion zwischen der extrazellulären Matrix und den Fibroblasten erhöht und/oder verbessert und der ausgewählt ist aus Ascorbinsäure und den Ascorbinsäurederivaten Natriumascorbylphosphat, Magnesiumascorbylphosphat, Ascorbylpalmitat, Dinatriumascorbylphosphat, Dinatriumascorbylsulfat, Natriumascorbat, Magnesiumascorbat, Ascorbylstearat, Ascorbyldipalmitat, Ascorbylacetat, Kaliumascorbyltocopherylphosphat, Chitosanascorbat und Ascorbylglucosid.

Weitere erfindungsgemäß bevorzugte Wirkstoffe, die die Interaktion zwischen der extrazellulären Matrix und den Fibroblasten erhöhen und/oder verbessern, sind ausgewählt aus Apfelkernextrakten (Pyrus Malus (Apple) Fruit Extract). Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.

Erfindungsgemäß besonders bevorzugte kosmetische oder dermatologische Zusammensetzungen sind dadurch gekennzeichnet, dass sie den Rohstoff Ederline in Mengen von 0,1 - 10 Gew.%, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Apfelkernextrakt in Mengen von 0,001 - 2 Gew.-%, bevorzugt 0,01 - 1,6 Gew.-% und besonders bevorzugt 0,03 - 1 Gew.%, jeweils bezogen auf den Gehalt an Aktivsubstanz in der gesamten Zusammensetzung, enthalten.

Weitere erfindungsgemäß bevorzugte Wirkstoffe, die die Interaktion zwischen der extrazellulären Matrix und den Fibroblasten erhöhen und/oder verbessern, sind ausgewählt aus Lotuskeim-Extrakten. Ein erfindungsgemäß besonders bevorzugter Lotuskeim-Extrakt ist unter der Handelsbezeichnung Lotus Germ Extract mit der INCl-Bezeichnung Water, Butylene Glycol, Nelumbo Nucifera Germ Extract von der Firma Maruzen erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus dem Dipeptid L-Citrullyl-L-arginin und seinen mit mindestens einer, bevorzugt linearen, C₂-C₂₂-Fettsäure N-acylierten und/oder veresterten Derivaten, insbesondere dem N-acetylierten Dipeptidderivat mit der INCI-Bezeichnung Acetyl Citrull Amido Arginine. Eine erfindungsgemäß besonders bevorzugte Zubereitung von Acetyl Citrull Amido Arginine ist unter der Handelsbezeichnung Exsy-Algine von der Firma Exsymol erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Rotweinextrakten (Wine Extract). Ein erfindungsgemäß besonders bevorzugter Rotweinextrakt ist unter der Handelsbezeichnung Sepivinol R von der Firma Seppic erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Traubenkernextrakten (Vitis Vinifera (Grape) Seed Extract), die besonders bevorzugt aus der Chardonnay-Traube stammen. Erfindungsgemäß besonders bevorzugte Traubenkernextrakte sind unter der Handelsbezeichnung Herbalia Grape von der Firma Cognis oder unter der Handelsbezeichnung Crodarom Chardonnay von Croda erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Schwarzen Holunderblüten (Sambucus Nigra Flower Extract). Ein erfindungsgemäß besonders bevorzugter Extrakt aus Schwarzen Holunderblüten ist unter der Handelsbezeichnung Sambucus AO von der Firma Alpaflor/ Centerchem bzw. von Permcos erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Hydroxystilbenen und deren Estern, insbesondere Resveratrol (trans-Stilben-3,4'-5-triol) und/oder Resveratrolmono-, -di- und -triphosphorsäureestern und deren Salzen, weiterhin aus Piceatannol, Plceatannolethern und Pinosylvin sowie Pinosylvinethern. Ein erfindungsgemäß besonders bevorzugter Resveratrolphosphorsäureester ist Trisodium Resveratrol Triphosphate, z. B. erhältlich von Ajinomoto.

Weitere erfindungsgemäß bevorzugte Wirkstoffe, die die Interaktion zwischen der extrazellulären Matrix und den Fibroblasten erhöhen und/oder verbessern, sind ausgewählt aus Keratinhydrolysaten, insbesondere Wollkeratinhydrolysaten. Ein erfindungsgemäß besonders bevorzugtes Wollkeratinhydrolysat ist unter der Bezeichnung Keratec Pep von der Firma Croda erhältlich. Keratec Pep weist eine kleinere Molmassenfraktion mit einer durchschnittlichen Molmasse von 150 Dalton und eine größere Molmassenfraktion mit einer durchschnittlichen Molmasse von 1265 Dalton auf.

Weitere erfindungsgemäß bevorzugte Wirkstoffe, die die Interaktion zwischen der extrazellulären Matrix und den Fibroblasten erhöhen und/oder verbessern, sind ausgewählt aus Conchiolinhydrolysaten. Erfindungsgemäß besonders bevorzugte Conchiolinhydrolysate sind unter den Bezeichnungen Pearl Protein Extract und Pearl Protein Extract BG von der Firma Maruzen erhältlich. Bei Conchiolinhydrolysaten handelt es sich, im Unterschied zu anderen auf dem Markt erhältlichen Perlenextrakten, nicht um Extrakte aus gemahlenen Perlen, wie sie z. B. unter der Bezeichung Crodarom Pearl mit der INCI-Bezeichnung Aqua (Water), Glycerin, Pearl Powder, Maris Sal (Sea Salt) von Croda erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet.

Ein weiterer erfindungsgemäß bevorzugter Wirkstoff, der die Interaktion zwischen der extrazellulären Matrix und den Fibroblasten erhöht und/oder verbessert, ist das Peptidderivat L-Glutamylaminoethyl-indol (erhältlich z. B. unter dem Handelsnamen Glistin von der Firma Exsymol).

Weitere erfindungsgemäß bevorzugte Wirkstoffe, die die Interaktion zwischen der extrazellulären Matrix und den Fibroblasten erhöhen und/oder verbessern, sind ausgewählt aus Isoflavonoiden und Isoflavonoid-reichen Pflanzenextrakten.
Zu den Isoflavonoiden zählen erfindungsgemäß die Isoflavone und die Isoflavon-Glycoside.
Unter Isoflavonen sind erfindungsgemäß Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Besonders bevorzugte Isoflavone sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Weiterhin ist es erfindungsgemäß bevorzugt, wenn die Isoflavone und/oder deren Glycoside als Bestandteile eines aus einer Pflanze gewonnenen Substanzgemisches, insbesondere eines pflanzlichen Extrakts, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Lipobelle Soyaglycone (Mibelle AG Cosmetics), Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß besonders bevorzugte kosmetische oder dermatologische Zusammensetzungen sind dadurch gekennzeichnet, dass sie mindestens ein Isoflavonoid in Gesamtmengen von 0,00001 bis 1 Gew.%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

Ein weiterer erfindungsgemäß bevorzugter Wirkstoff, der die Interaktion zwischen der extrazellulären Matrix und den Fibroblasten erhöht und/oder verbessert, ist Dihydroquercetin (3,3',4',5,7-Pentahydroxyflavanon), das auch als Taxifolin bezeichnet wird.

Erfindungsgemäß besonders bevorzugte kosmetische oder dermatologische Zusammensetzungen sind dadurch gekennzeichnet, dass sie mindestens einen Wirkstoff, der die Interaktion zwischen der extrazellulären Matrix und den Fibroblasten erhöht und/oder verbessert, in einer Gesamtmenge von 0,000001 - 10 Gew.-%, bevorzugt 0,00001 - 5 Gew.-%, besonders bevorzugt 0,0001 - 2 Gew.-% und außerordentlich bevorzugt 0,005 - 0,5 oder 1 Gew.-%, enthalten, jeweils bezogen auf den Gehalt an Aktivsubstanz in der gesamten Zusammensetzung.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen kosmetischen Wirkstoff enthalten, der ausgewählt ist aus :
- Monomeren und Oligomeren von Aminosäuren, N-C₂-C₂₄-Acylaminosäuren, den Estern und/oder den physiologisch verträglichen Metallsalzen dieser Substanzen, die nicht die Degranulierung von Mastzellen und/oder die Ausschüttung von Histamin reduzieren,
- DNA-Reparaturenzymen,
- DNA- oder RNA-Oligonucleotiden,
- Vitaminen, Provitaminen und Vitaminvorstufen der Gruppen B, E, H und K und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten, die nicht die Degranulierung von Mastzellen und/oder die Ausschüttung von Histamin reduzieren können,
- Ubichinon und Ubichinol sowie deren Derivaten,
- Silymarin,
- Ectoin,
- anorganischen und organischen UV-Filtersubstanzen,
- selbstbräunenden,
- hautberuhigenden,
- feuchtigkeitsspendenden und
- sebumregulierenden Wirkstoffen sowie
- Mischungen dieser Wirkstoffe.
   Die Monomere der Aminosäuren und/oder der N-C₂-C₂₄-Acylaminosäuren, die nicht die Degranulierung von Mastzellen und/oder die Ausschüttung von Histamin reduzieren können, sind bevorzugt ausgewählt aus Alanin, Arginin, Asparagin, Asparaginsäure, Canavanin, Citrullin, Cystein, Cystin, Desmosin, Glutamin, Glutaminsäure, Glycin, Histidin, Homophenylalanin, Hydroxylysin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Taurin, Zink- und Natriumpyroglutamat und Natriumlauroylglutamat.
   Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.

Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Die Oligomere der Aminosäuren und/oder der N-C₂-C₂₄-Acylaminosäuren, die nicht die Degranulierung von Mastzellen und/oder die Ausschüttung von Histamin reduzieren, sind bevorzugt ausgewählt aus Di-, Tri-, Tetra-, Penta-, Hexa- oder Pentadecapeptiden, die N-acyliert und/oder verestert sein können. Erfindungsgemäß bevorzugte, gegebenenfalls N-acylierte und/oder veresterte Dipeptide sind Val-Trp (Dipeptide-2), Asn-Phe, Asp-Phe, N-Palmitoyl-β-Ala-His, Carnosin (β-Ala-His) und N-Palmitoyl-Pro-Arg. Erfindungsgemäß besonders bevorzugte, gegebenenfalls N-acylierte und/oder veresterte Tripeptide sind Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn und N-Acetyl-Arg-Lys-Arg-NH₂. Erfindungsgemäß bevorzugte, gegebenenfalls N-acylierte und/oder veresterte Pentapeptide N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Val-Val-Arg-Pro-Pro-Pro, Ala-Arg-His-Methylnorleucin-Homophenylalanin-Trp (Hexapeptide-7) und Hexapeptide-11 (z. B. Peptamide-6 von Arch Personal Care). Ein erfindungsgemäß besonders bevorzugtes Pentadecapeptid ist z. B. der Rohstoff Vinci 01 von Vincience (Pentadecapeptide-1).

Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als so genannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.
Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.
Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosomes^{™} (INCl-Bezeichnung: Water, Lecithine, Plankton Extract) mit einer Proteinkonzentration von 0,25 - 2 mg/ml, liposomenverkapselte T4N5 z. B. unter der Bezeichnung Ultrasomes^{™} (INCl-Bezeichnung: Water, Lecithine, Micrococcus Luteus Extract) mit einer Proteinkonzentration von 2 - 5 mg/ml (gemessen mit dem Bradford-Assay) von AGI Dermatics, USA, erhältlich.
Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen kosmetischen Wirkstoff, der ausgewählt ist aus Photosomes^{™} und/oder Ultrasomes^{™}, in Gesamtmengen von 0,1 - 10 Gew.-%, bevorzugt 0,5 - 5,0 Gew.-% und besonders bevorzugt 1,0 - 4,0 Gew.-%, bezogen auf das gesamte Mittel, enthalten.
Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen kosmetischen Wirkstoff, der ausgewählt ist aus den Monomeren und Oligomeren von Aminosäuren, N-C₂-C₂₄-Acylaminosäuren und/oder den Estern und/ oder den physiologisch verträglichen Metallsalzen dieser Substanzen, die nicht die Degranulierung von Mastzellen und/oder die Ausschüttung von Histamin reduzieren können, in Gesamtmengen von 0,00001 - 10 Gew.-%, bevorzugt 0,001 - 5 Gew.-% und besonders bevorzugt 0,005 - 3 Gew.-%, jeweils bezogen auf die gesamte Zusammensetzung, enthalten.

In einer weiteren bevorzugten Ausführungsform der Erfindung liegen die Monomeren und Oligomeren von Aminosäuren, N-C₂-C₂₄-Acylaminosäuren, den Estern und/oder den physiologisch verträglichen Metallsalzen dieser Substanzen in geträgerter Form vor, insbesondere aufgetragen auf feinteiligen, pulverförmigen Substraten wie Kieselgel, insbesondere Aerosil-Typen, weiterhin Talkum, Microsponges, modifizierten Stärken und Stärkederivaten, kristalliner Cellulose, Cellulosepulvern, Lactoglobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen kosmetischen Wirkstoff enthalten, der ausgewählt ist aus DNA-Oligonucleotiden und RNA-Oligonucleotiden.

Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glycosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).
Ein erfindungsgemäß besonders bevorzugtes Oligonucleotid ist das Thymidin-Dinucleotid.
In den besonders bevorzugten erfindungsgemäßen Zusammensetzungen sind die DNA-Oligonucleotide oder RNA-Oligonucleotide in Mengen von 0,00001 - 5 Gew.-%, bevorzugt 0,0001 - 1,0 Gew.-% und besonders bevorzugt 0,0005 - 0,5 Gew.-%, bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen kosmetischen Wirkstoff enthalten, der ausgewählt ist aus mindestens einem Vitamin, Provitamin oder einer als Vitaminvorstufe bezeichnete Verbindung aus den Vitamingruppen B, E, H und K und den Estern der vorgenannten Substanzen.
Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.%, bezogen auf die gesamte Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,05 bis 1 Gew.%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder

Panthenol auch Derivate des 2-Furanon mit der allgemeinen Strukturformel (VIT-I) eingesetzt werden.

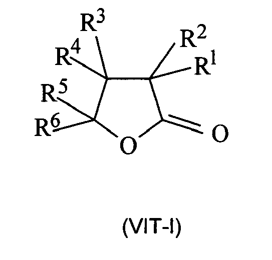

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl-oder Hydroxymethylrest, einen gesättigten oder ein- oder zweifach ungesättigten, linearen oder verzweigten C₂-C₄- Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.%, besonders bevorzugt 0,5 bis 2 Gew.%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3aS,4S, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.%, insbesondere 0,01 - 0,5 Gew.-%, bezogen auf die Zusammensetzung, enthalten.

In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und/oder Salzen und aus Pantolacton.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.%, jeweils bezogen auf die gesamte Zusammensetzung, enthalten.
Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen kosmetischen Wirkstoff enthalten, der ausgewählt ist aus mindestens einer α-Hydroxycarbonsäure, α-Ketocarbonsäure oder β-Hydroxycarbonsäure oder deren Ester-, Lacton- und/ oder Salzform. Überraschend wurde festgestellt, dass die anti-irritierende Wirkung des Wirkstoffs b), der die Degranulierung von Mastzellen und/oder die Ausschüttung von Histamin reduziert, in Gegenwart der ebenfalls als die Haut reizend bekannten α-Hydroxycarbonsäuren, α-Ketocarbonsäuren oder β-Hydroxycarbonsäuren uneingeschränkt zur Geltung kommt und dass unter bestimmten Umständen durch einen derartigen Zusatz die Anticellulite-Wirkung des hyperämisierenden Wirkstoffs weiter gesteigert werden kann.
Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer- und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und Calcium-Salze.
Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.%, besonders bevorzugt 0,5 - 5 Gew.-% und außerordentlich bevorzugt 1 - 2 Gew.-%, jeweils bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen kosmetischen Wirkstoff enthalten, der ausgewählt ist aus mindestens einem Flavonoid und/oder mindestens einem Flavonoid-reichen Pflanzenextrakt, der nicht die Degranulierung von Mastzellen und/oder die Ausschüttung von Histamin reduziert.
Die erfindungsgemäß bevorzugten Flavonoide umfassen die Glycoside der Flavone, der 3-Hydroxyflavone (Flavonole) und der Aurone. Die Flavanone und die Isoflavone sind erfindungsgemäß ausdrücklich von den Flavonoiden ausgenommen. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-a-L-mannopyranosyl)-β-D-glucopyranosid)), Monoxerutin (3,3',4',5-Tetrahydroxy-7-(2-hydroxyethoxy)flavon-3-(6-O-(6-deoxy-a-L-mannopyranosyl)-β-D-glucopyranosid)), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.
Erfindungsgemäß bevorzugt werden die Flavonoide in Mengen von 0,0001 bis 1 Gew.-%, besonders bevorzugt 0,0005 bis 0,5 Gew.-% und außerordentlich bevorzugt 0,001 bis 0,1 Gew.-%, jeweils bezogen auf die Flavonoidaktivsubstanz in der gesamten kosmetischen Zusammensetzung, eingesetzt.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen kosmetischen Wirkstoff enthalten, der ausgewählt ist aus mindestens einem Ubichinon oder einem Ubichinol oder deren Derivaten. Ubichinole sind die reduzierte Form der Ubichinone. Die erfindungsgemäß bevorzugten Ubichinone weisen die Formel (UBI-I) auf: 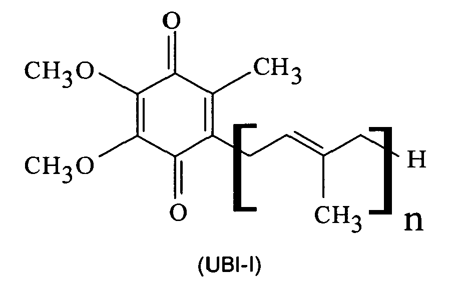 mit n = 6, 7, 8, 9 oder 10.
Besonders bevorzugt ist das Ubichinon der Formel (UBI-I) mit n = 10, auch bekannt als Coenzym Q10.
Erfindungsgemäß bevorzugt werden die Ubichinone, Ubichinole oder deren Derivate in einer Gesamtmenge von 0,0001 - 1 Gew.%, besonders bevorzugt 0,001 - 0,5 Gew.-% und außerordentlich bevorzugt 0,005 - 0,1 Gew.-%, jeweils bezogen auf die gesamte Zusammensetzung, eingesetzt.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin Silymarin enthalten. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.
Erfindungsgemäß bevorzugt wird Silymarin in einer Gesamtmenge von 0,0001 - 1 Gew.-%, besonders bevorzugt 0,001 - 0,5 Gew.-% und außerordentlich bevorzugt 0,005 - 0,1 Gew.-%, jeweils bezogen auf die gesamte Zusammensetzung, eingesetzt.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin Ectoin enthalten. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.%, jeweils bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens eine anorganische und/oder mindestens eine organische UV-Filtersubstanz enthalten.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.
Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/ Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCl: Bis-Ethylhexyloxyphenol Methoxyphenyl Triazin, unter dem Namen Tinosorb S bei CIBA erhältlich), Dioctylbutylamidotriazon (INCl: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1 (di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCl: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'-methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.
Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Das Coatingmaterial kann aber auch anorganische Materialien umfassen; besonders bevorzugt ist Silicoiumdioxid als Coatingmaterial. Besonders bevorzugte Pigmentmaterialien sind Titandioxid und Zinkoxid.
Erfindungsgemäß sind die organischen UV-Filtersubstanzen bevorzugt in Mengen von 0,1 - 30 Gew.%, besonders bevorzugt 0,5 - 20 Gew.%, außerordentlich bevorzugt 1,0 - 15 Gew.-% und weiter bevorzugt 3,0 - 10 Gew.-%, jeweils bezogen auf die gesamte Zusammensetzung, enthalten. Erfindungsgemäß sind die anorganischen UV-Filtersubstanzen bevorzugt in Mengen von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen selbstbräunenden Wirkstoff enthalten. Erfindungsgemäß bevorzugte selbstbräunende Wirkstoffe sind ausgewählt aus Dihydroxyaceton, Erythrulose und 5,6-Dihydroxyindolin, besonders bevorzugt aus Kombinationen von Dihydroxyaceton und Erythrulose.
Erfindungsgemäß bevorzugt ist mindestens ein selbstbräunender Wirkstoff in einer Gesamtmenge von 0,01 - 15 Gew.%, besonders bevorzugt 0,1 - 10 Gew.%, außerordentlich bevorzugt 1,0 - 5 Gew.-% und weiterhin außerordentlich bevorzugt 2,0 - 4,0 Gew.-%, jeweils bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen hautberuhigenden Wirkstoff enthalten. Erfindungsgemäß bevorzugte hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCl-Bezeichnung "Sorbitol, Yeast Extract"), erhältlich von der Firma Lanatech, den physiologisch verträglichen Salzen von Sterolsulfaten, wie sie z. B. unter dem Handelsnamen Phytocohesine (INCl: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, sowie beliebigen Mischungen dieser Substanzen.
Der mindestens eine hautberuhigende Wirkstoff ist bevorzugt in einer Gesamtmenge von 0,001 - 5 Gew.-%, besonders bevorzugt 0,01 - 2 Gew.-% und außerordentlich bevorzugt 0,1 - 1 Gew.-%, jeweils bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen feuchtigkeitsspendenden Wirkstoff enthalten. Erfindungsgemäß bevorzugte feuchtigkeitsspendende Wirkstoffe sind ausgewählt aus Desoxyzuckern, besonders bevorzugt Rhamnose und Fucose, Desoxyzucker-Bausteine enthaltende Polysaccharide, besonders bevorzugt Biosaccharide Gum-1, enthalten beispielsweise in dem Handelsprodukt Fucogel^{®} von Solabia Biosaccharide Gum-2, enthalten beispielsweise in dem Handelsprodukt Rhamnosoft^{®} von Solabia, Biosaccharide Gum-3, enthalten beispielsweise in dem Handelsprodukt Fucogenol^{®} von Solabia, Biosaccharide Gum-4, enthalten beispielsweise in dem Handelsprodukt Glycofilm^{®} von Solabia, Mischungen aus Biosaccharide Gum-2 und Biosaccharide Gum-3, beispielsweise erhältlich als Handelsprodukt Elastinol plus^{®} von Solabia, weiterhin Harnstoff, Bis-N,N'-(2-Hydroxyethyl)harnstoff, Betain (Me₃N⁺-CH₂-COO⁻), Glycosaminoglycanen, besonders bevorzugt Hyaluronsäure und deren Salze und Silanolester, Dextran, Dextransulfat, Chondroitin-4-sulfat, Chondroitin-6-sulfat, Mischungen von Bis-N,N'-(2-Hydroxyethyl)harnstoff und Harnstoff und besonders bevorzugt Mischungen von Bis-N,N'-(2-Hydroxyethyl)harnstoff, Harnstoff und Milchsäure(salzen).

Der mindestens eine feuchtigkeitsspendende Wirkstoff ist bevorzugt in einer Gesamtmenge von 0,001 - 10 Gew.%, besonders bevorzugt 0,01 - 5 Gew.-% und außerordentlich bevorzugt 0,1 - 1 oder 2 Gew.-%, jeweils bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen sebumregulierenden Wirkstoff enthalten. Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von Seppic), Chrysanthemenextrakt (z. B. Laricyl^{®} von Laboratoires Sérobiologiques) und den Wirkstoffmischungen Asebiol^{®} BT 2 (Laboratoires Sérobiologiques, INCl: Aqua, Hydrolyzed Yeast Protein, Pyridoxine, Niacinamide, Glycerin, Panthenol, Allantoin, Biotin) und Antifettfaktor^{®} COS-218/2-A (Cosmetochem, INCl: Aqua, Cetyl-PCA, PEG-8 Isolauryl Thioether, PCA, Cetyl Alcohol).
Der mindestens eine sebumregulierende Wirkstoff ist bevorzugt in einer Gesamtmenge von 0,001 - 5 Gew.-%, besonders bevorzugt 0,01 - 2 Gew.-% und außerordentlich bevorzugt 0,1 bis 1 Gew.-%, jeweils bezogen auf die gesamte Zusammensetzung, enthalten.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, gemäß einem der Patentansprüche 1 - 11 enthält, zur Behandlung der Cellulite und/oder zur Steigerung der Hautfestigkeit und/oder zur Verbesserung der mechanischen Stabilität der Haut und/oder zur Steigerung der Hautelastizität und/oder zur Straffung der Haut und/oder zur Glättung der Haut und/oder zur Verminderung von Falten, Fältchen und/oder feinen Linien und/oder zur Verbesserung des optischen Erscheinungsbildes der Haut.

Ein weiterer Gegenstand der vorliegenden Erfindung ist die Verwendung einer Wirkstoffkombination aus a) mindestens einem hyperämisierenden Wirkstoff und b) mindestens einem Wirkstoff, der die Degranulierung von Mastzellen und/oder die Ausschüttung von Histamin reduziert, zur Herstellung einer kosmetischen oder dermatologischen Zusammensetzung
- zur Behandlung der Cellulite und/oder
- zur Steigerung der Hautfestigkeit und/oder
- zur Verbesserung der mechanischen Stabilität der Haut und/oder
- zur Steigerung der Hautelastizität und/oder
- zur Straffung der Haut und/oder
- zur Glättung der Haut und/oder
- zur Verminderung von Falten, Fältchen und/oder feinen Linien und/oder
- zur Verbesserung des optischen Erscheinungsbildes der Haut.

Ein weiterer Gegenstand der vorliegenden Erfindung ist die Verwendung mindestens eines Wirkstoffs, der die Degranulierung von Mastzellen und/oder die Ausschüttung von Histamin reduziert, zur Verbesserung der Hautverträglichkeit von Hautbehandlungsmitteln mit mindestens einem hyperämisierenden Wirkstoff, insbesondere von Anticellulitemitteln. Für eine derartige erfindungsgemäße Verwendung besonders bevorzugte Wirkstoffe, die die Degranulierung von Mastzellen und/oder die Ausschüttung von Histamin reduzieren können, sind ausgewählt aus
- Extrakten aus den Samen von Cucurbita pepo (Zucchini),
- Extrakten aus Mentha piperita, insbesondere aus den Blättern von Mentha piperita,
- N-acylierten Alkanolaminen, insbesondere N-Palmitoylethanolamin,
- Hydrolysaten oder Extrakten aus der Alge *Enteromorpha compressa* (Darmtang, eine Grünalge), insbesondere aus dem Vegetationskörper (Thallus) der Alge, z. B. in Enteline 2,
- dem Dipeptid Tyr-Arg, dessen Estern und dessen N-Acyl-Derivaten, insbesondere N-Acetyl-Tyr-Arg-hexyldecylester (z. B. Calmosensine von Sederma),
- sowie Mischungen hiervon.

Erfindungsgemäß besonders bevorzugte kosmetische oder dermatologische Zusammensetzungen zur topischen Behandlung der Haut, die in einem geeigneten kosmetischen oder dermatologischen Träger a) mindestens einen hyperämisierenden Wirkstoff und b) mindestens einen Wirkstoff, der die Degranulierung von Mastzellen und/oder die Ausschüttung von Histamin reduziert, enthalten, sind dadurch gekennzeichnet, dass sie weiterhin mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.
Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆₋₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 -10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.
Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte Zusammensetzung.

Bevorzugt liegen die erfindungsgemäßen kosmetischen oder dermatologischen Zusammensetzungen in Form einer flüssigen, fließfähigen, sprühfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaums, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.
In einer besonders bevorzugten Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.
In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospholipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn - Salze.
Die erfindungsgemäßen Zusammensetzungen enthalten die Emulgatoren bevorzugt in Mengen von 0,1 - 25 Gew.%, besonders bevorzugt 0,5 - 15 Gew.-%, bezogen auf die gesamte Zusammensetzung.
In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat. Die Anwesenheit von Emulgatoren der allgemeinen Formel R¹ - O - R² kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher dadurch gekennzeichnet, dass sie in Form einer lamellare Strukturen aufweisenden Öl-in-Wasser-Emulsion vorliegen.
In einer weiteren besonders bevorzugten Ausführungsform liegen die erfindungsgemäßen Zusammensetzungen in Form einer Wasser-in-Öl-Emulsion vor. Derartige Wasser-in-Öl-Emulsionen können so formuliert werden, dass die eine höhere Viskosität aufweisen, so dass sie sorgfältig in die Haut einzumassieren sind. Dies unterstützt die Anticellulite-Wirkung der erfindungsgemäßen Zusammensetzungen. Genau so gut lassen sich aber auch niedrig-viskose Wasser-in-Öl-Emulsionen formulieren, insbesondere auf Basis des polymeren Wasser-in-Öl-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-Öl-Emulsionen formulieren.
Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.
Weitere bevorzugte Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

Da die erfindungsgemäßen Zusammensetzungen insbesondere für die (großflächige) Anwendung auf der Körperhaut bestimmt sind, ist es besonders vorteilhaft und praktisch, wenn sie als sprühbare Zusammensetzung vorliegen. Ein weiterer Gegenstand der vorliegenden Erfindung ist daher eine Zusammensetzung gemäß einem der Ansprüche 1 - 10, die in einem Sprühspender oder Pumpspender verpackt ist.

Die nachfolgenden Formulierungsbeispiele sollen den Gegenstand der Erfindung erläutern, ohne ihn hierauf zu beschränken.

Alle Mengenangaben sind in Gew.%, bezogen auf die gesamte Zusammensetzung.

### 1. Oel-in-Wasser-Emulsionen

### 1. Beispielserie:

Die Zusammensetzungen 1.1 - 1.3 sind bevorzugte Ausführungsformen von pflegenden Tagescremes mit einem Anticellulite-Effekt bzw. einem Effekt zur Verbesserung der Silhouette des Gesichts, insbesondere im Kinn-Bereich, mit erfindungsgemäß verbesserter Hautverträglichkeit.

| | 1.1 | 1.2 | 1.3 |
|---|---|---|---|
| Cetiol SN | 4,00 | 4,00 | 4,00 |
| Mineralöl | 6,00 | 6,00 | 6,00 |
| Cutina CBS | 2,00 | 2,00 | 2,00 |
| Edenor L2 SM | 1,50 | 1,50 | 1,50 |
| Eumulgin B3 | 1,00 | 1,00 | 1,00 |
| Baysilon M 350 | 1,00 | 1,00 | 1,00 |
| Tocopherylacetat | 0,50 | 0,50 | 0,50 |
| Propylparaben | 0,30 | 0,30 | 0,30 |
| Mandelöl | 2,00 | 2,00 | 2,00 |
| Pemulen TR-1 | 0,30 | 0,30 | 0,30 |
| Glycerin | 5,00 | 3,00 | 3,00 |
| Milchsäure 80% | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Panthenol | 0,50 | 0,50 | 0,50 |
| Sepigel 305 | 0,50 | 0,50 | 0,50 |
| Silk Protein | 0,25 | 0,25 | 0,25 |
| Keltrol SF | 0,20 | 0,20 | 0,20 |
| Hotact VBE | 0,10 | 0,25 | 0,35 |
| Ocaline | - | - | 3,00 |
| N-Palmitoylethanolamin | 0,50 | - | - |
| Calmiskin | - | 1,50 | - |
| Parfum | 0,30 | 0,30 | 0,30 |
| Aqua | ad 100 | ad 100 | ad 100 |

### 2. Beispielserie:

Die Zusammensetzungen 2.1 - 2.3 sind bevorzugte Ausführungsformen von pflegenden Cremes mit gelähnlicher konsistenz mit einem Anticellulite-Effekt bzw. einem Effekt zur Verbesserung der Silhouette des Gesichts, insbesondere im Kinn-Bereich, mit erfindungsgemäß verbesserter Hautverträglichkeit.

| | 2.1 | 2.2 | 2.3 |
|---|---|---|---|
| 2-Ethylhexylpalmitat | 2,00 | 2,00 | 2,00 |
| Dow Corning 1501 Fluid | 5,00 | 5,00 | 5,00 |
| Synperonic PE L 64 | 2,00 | 2,00 | 2,00 |
| Dipropylenglycol | 5,00 | 5,00 | 5,00 |
| Tego Carbomer 140 | 0,70 | 0,70 | 0,70 |
| Calcium D-Panthothenat | 0,05 | 0,05 | 0,05 |
| Keratolite | 1,00 | 1,00 | 1,00 |
| Simulgel NS | 0,25 | 0,25 | 0,25 |
| Parfum | 0,30 | 0,30 | 0,30 |
| Hotact VBE | 0,10 | 0,25 | 0,05 |
| Ethylnicotinat | 0,10 | - | - |
| Ocaline | 2,00 | - | 3,00 |
| Enteline 2 | - | 2,00 | - |
| Aqua | ad 100 | ad 100 | ad 100 |

### 3. Beispielserie:

Die Zusammensetzungen 3.1 - 3.3 sind bevorzugte Ausführungsformen von Tagescremes für besonders anspruchsvolle Haut mit einem Anticellulite-Effekt bzw. einem Effekt zur Verbesserung der Silhouette des Gesichts, insbesondere im Kinn-Bereich, mit erfindungsgemäß verbesserter Hautverträglichkeit und einem Gehalt an Antiageing-Wirkstoffen.

| | 3.1 | 3.2 | 3.3 |
|---|---|---|---|
| Lipoid S75-3 | 0,50 | 0,50 | 0,50 |
| Isopropylstearat | 4,00 | 4,00 | 4,00 |
| Cetiol B | 2,00 | 2,00 | 2,00 |
| Tocopherylacetat | 0,50 | 0,50 | 0,50 |
| Cutina MD | 1,00 | 1,00 | 1,00 |
| Lanette 22 | 2,00 | 2,00 | 2,00 |
| Baysilone M 350 | 0,50 | 0,50 | 0,50 |
| Propylparaben | 0,20 | 0,20 | 0,20 |
| Dow Corning 9040 | 1,00 | 1,00 | 1,00 |
| Glycerin | 4,50 | 3,00 | 3,00 |
| Hexandiol | 6,00 | 3,00 | |
| Methylparaben | 0,20 | 0,20 | 0,20 |
| Tego Carbomer 140 | 0,30 | 0,30 | 0,30 |
| DSH-CN | 5,00 | 5,00 | 5,00 |
| Photosomes | 0,20 | 0,20 | 0,20 |
| Lipochroman-6 | 0,01 | 0,01 | 0,01 |
| Propylenglycol | 5,00 | 5,00 | 5,00 |
| Matrixyl 3000 | - | 3,00 | - |
| Simulgel NS | 1,50 | 1,50 | 1,50 |
| TiO₂ | 0,50 | 0,50 | - |
| Hotact VBE | - | 0,20 | 0,35 |
| N-Vanillylnonamide | 0,20 | - | - |
| Ocaline | - | - | 3,00 |
| N-Palmitoylethanolamin | 0,50 | - | - |
| Caomint | - | 2,00 | - |
| Parfum | 0,35 | 0,35 | 0,35 |
| Aqua | ad 100 | ad 100 | ad 100 |

### 4. Beispielserie:

Die Zusammensetzungen 4.1 - 4.5 sind bevorzugte Ausführungsformen von Tagescremes mit höherem Lichtschutzfilter (SPF ca. 10 - 15) für besonders anspruchsvolle Haut mit einem Anticellulite-Effekt bzw. einem Effekt zur Verbesserung der Silhouette des Gesichts, insbesondere im Kinn-Bereich, mit erfindungsgemäß verbesserter Hautverträglichkeit und einem Gehalt an Antiageing-Wirkstoffen.

| | 4.1 | 4.2 | 4.3 | 4.4 | 4.5 |
|---|---|---|---|---|---|
| Montanov 68 | 5,00 | 5,00 | 5,00 | 5,00 | 5,00 |
| Myritol318 | 5,00 | 5,00 | 5,00 | 5,00 | 5,00 |
| Cetiol SB 45 | 0,50 | 0,50 | 0,50 | 0,50 | 0,50 |
| Novata AB | 2,00 | 2,00 | 2,00 | 2,00 | 2,00 |
| Stenol 16/18 | 1,00 | 1,00 | 1,00 | 1,00 | 1,00 |
| Baysilon M 350 | 0,50 | 0,50 | 0,50 | 0,50 | 0,50 |
| Tocopherylacetat | 0,50 | 0,50 | 0,50 | 0,50 | 0,50 |
| Controx KS | 0,25 | 0,25 | 0,25 | 0,25 | 0,25 |
| Parsol SLX | - | 4,00 | 4,00 | - | - |
| Parsol HS | - | 2,00 | 2,00 | 2,00 | - |
| Neo Heliopan AP | - | - | - | 1,00 | - |
| Parsol 340 | - | - | - | 5,00 | - |
| Uvinul MBC 95 | 2,00 | - | - | - | - |
| Parsol 1789 | 1,00 | 1,80 | 1,80 | - | - |
| Propylparaben | 0,20 | 0,20 | 0,20 | 0,20 | 0,20 |
| Tego Carbomer 140 | 0,50 | 0,50 | 0,50 | 0,50 | 0,50 |
| 1,6-Hexandiol | 6,00 | 6,00 | 3,00 | 6,00 | - |
| Talkum | 0,50 | 0,50 | 0,50 | 0,50 | 0,50 |
| Methylparaben | 0,20 | 0,20 | 0,20 | 0,20 | 0,20 |
| Glycerin | 4,50 | 4,50 | 3,00 | 4,50 | 3,00 |
| Dry Flo Plus | 1,00 | 1,00 | 1,00 | 1,00 | 1,00 |
| Natipide 2 PG | 1,00 | 1,00 | 1,00 | 1,00 | 1,00 |
| Phytokine | 5,00 | 5,00 | 5,00 | 5,00 | 5,00 |
| Phenoxyethanol | 0,40 | 0,40 | 0,40 | 0,40 | 0,40 |
| Hotact VBE | 0,10 | 0,25 | 0,35 | 0,50 | - |
| Calmosensine | - | 2,00 | - | - | - |
| Ocaline | 1,00 | - | 3,00 | 4,00 | 2,00 |
| Herbasol Extract Capsicum | - | - | - | - | 0,50 |
| Parfum | 0,40 | 0,40 | 0,40 | 0,40 | 0,40 |
| Aqua | ad 100 | ad 100 | ad 100 | ad 100 | ad 100 |

### 5. Wasser-in-Öl-Emulsion

Die Zusammensetzungen 5.1 - 5.3 sind bevorzugte Ausführungsformen von Cremes mit einem Anticellulite-Effekt bzw. einem Effekt zur Verbesserung der Silhouette des Gesichts, insbesondere im Kinn-Bereich, mit erfindungsgemäß verbesserter Hautverträglichkeit, auf der Basis einer reichhaltigen Wasser-in-Öl-Emulsion, die in die Haut einmassiert werden kann und dadurch einen zusätzlichen Bonuseffekt hervorruft.

| | 5.1 | 5.2 | 5.3 |
|---|---|---|---|
| Lameform TGI | 3,00 | 3,00 | 3,00 |
| Elfacos ST 37 | 0,50 | 0,50 | 0,50 |
| Microcrystalline Wax | 3,00 | 3,00 | 3,00 |
| Softisan 649 | 1,00 | 1,00 | 1,00 |
| Paraffinöl | 8,00 | 8,00 | 8,00 |
| Vaseline | 2,00 | 2,00 | 2,00 |
| Vitamin E Acetat | 2,00 | 2,00 | 2,00 |
| Methylparaben | 0,30 | 0,30 | 0,30 |
| Propylparaben | 0,30 | 0,30 | 0,30 |
| Isopropylisostearat | 8,00 | 8,00 | 8,00 |
| Glycerin | 5,00 | 5,00 | 5,00 |
| Hotact VBE | 0,10 | 0,25 | 0,35 |
| Ocaline | - | 2,00 | - |
| N-Palmitoylethanolamin | 0,25 | - | 0,50 |
| Milchsäure 80%ig | 0,60 | 0,60 | 0,60 |
| Panthenol | 0,5 | 1,0 | 0,5 |
| Calendula Officinalis Flower Extract | 0,3 | - | - |
| Propylenglycol | 0,5 | - | - |
| Parfum | 0,2 | 0,2 | 0,2 |
| Aqua | ad 100 | ad 100 | ad 100 |

### 6. Gesichtswasser

Die Zusammensetzungen 6.1 - 6.3 sind bevorzugte Ausführungsformen von Gesichtswässern mit einem straffenden Effekt zur Verbesserung der Silhouette des Gesichts und der Ausstrahlung des Teints, insbesondere bei müder Haut, mit erfindungsgemäß verbesserter Hautverträglichkeit.

| | 6.1 | 6.2 | 6.3 |
|---|---|---|---|
| Dipropylenglycol | 10,00 | 10,00 | 10,00 |
| Chlorhexidindigluconat | 1,00 | 1,00 | 1,00 |
| Synperonic PE7 L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil / Trideceth 9 / Propylen Glycol | 0,50 | 0,50 | 0,50 |
| Chlorella Vulgaris Extract | 0,50 | 0,50 | 0,50 |
| Hotact VBE | - | 0,25 | 0,35 |
| Herbasol Extrakt Capsicum | 1,00 | - | - |
| Ocaline | 1,00 | - | 2,00 |
| N-Palmitoylethanolamin | - | 0,50 | - |
| Caospice | 1,00 | - | - |
| Parfum | 0,20 | 0,20 | 0,20 |
| Aqua | ad 100 | ad 100 | ad 100 |

### 7. Wasser-in-Silicon-Emulsionen

Die Zusammensetzungen 7.1 - 7.3 sind bevorzugte Ausführungsformen von Cremes mit einem Anticellulite-Effekt bzw. einem Effekt zur Verbesserung der Silhouette des Gesichts, insbesondere im Kinn-Bereich, mit erfindungsgemäß verbesserter Hautverträglichkeit, auf der Basis einer reichhaltigen und dennoch nicht-fettenden Wasser-in-Silicon-Emulsion, die in die Haut einmassiert werden kann.

| | 7.1 | 7.2 | 7.3 |
|---|---|---|---|
| Belsil DM 100 | 2,50 | 2,50 | 2,50 |
| Dow Corning 245 Fluid | 25,00 | 25,00 | 25,00 |
| Abil EM 90 | 2,00 | 2,00 | 2,00 |
| Natriumchlorid | 2,00 | 2,00 | 2,00 |
| Parfum | 0,30 | 0,30 | 0,30 |
| Symdiol 68 | 0,30 | 0,30 | 0,30 |
| Phenoxyethanol | 0,40 | 0,40 | 0,40 |
| Hotact VBE | 0,35 | - | - |
| Pseudocapsaicin | - | 0,20 | - |
| Herbasol Capsicum Extrakt | - | - | 1,00 |
| Calmiskin | 2,00 | - | - |
| Ocaline | - | 3,00 | - |
| N-Palmitoylethanolamin | - | - | 0,50 |
| Aqua | ad 100 | ad 100 | ad 100 |

### 8. Beispielserie:

Die Zusammensetzungen 8.1 - 8.4 sind bevorzugte Ausführungsformen von Tagescremes für besonders anspruchsvolle Haut mit einem Anticellulite-Effekt bzw. einem Effekt zur Verbesserung der Silhouette des Gesichts, insbesondere im Kinn-Bereich, mit erfindungsgemäß verbesserter Hautverträglichkeit und einem erhöhten Gehalt an diversen Antiageing-Wirkstoffen.

| | 8.1 | 8.2 | 8.3 | 8.4 |
|---|---|---|---|---|
| Distelöl | 3,00 | 3,00 | 3,00 | 3,00 |
| Myritol318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Baysilone-Öl M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Algenextrakt | 1,00 | - | - | - |
| Symdiol 68 | 0,30 | 0,50 | - | - |
| DSH-CN | 5,00 | 5,00 | 5,00 | 5,00 |
| Hydrovance | 4,30 | 2,50 | 8,60 | 10,00 |
| Kombuchka | 3,00 | - | - | 3,00 |
| Glistin | - | 2,00 | - | - |
| Natrulon RC 50 DG | - | - | 1,00 | - |
| Phytokine | 2,00 | | 2,00 | 2,00 |
| Matrixy13000 | - | 2,00 | - | - |
| Olea europ. Fol extr. S. sicc. | - | - | - | 0,10 |
| Hotact VBE | 0,35 | - | - | - |
| Pseudocapsaicin | - | 0,20 | - | - |
| Herbasol Capsicum Extrakt | - | - | 1,00 | - |
| Caomint | 1,00 | 0,50 | 2,00 | - |
| Calmosensine | 1,00 | 2,00 | - | 2,00 |
| Calmiskin | 2,00 | - | - | - |
| Ocaline | - | 3,00 | - | - |
| N-Palmitoylethanolamin | - | - | 0,50 | - |
| Perfume | 0,10 | 0,10 | 0,10 | 0,10 |
| Aqua | ad 100 | ad 100 | ad 100 | ad 100 |

### 9. Hautcremes:

Die Zusammensetzungen 9.1 - 9.3 sind weitere bevorzugte Ausführungsformen von Cremes für besonders anspruchsvolle Haut mit einem Anticellulite-Effekt bzw. einem Effekt zur Verbesserung der Silhouette des Gesichts, insbesondere im Kinn-Bereich, mit erfindungsgemäß verbesserter Hautverträglichkeit und einem erhöhten Gehalt an diversen Antiageing-Wirkstoffen.

| | 9.1 | 9.2 | 9.3 |
|---|---|---|---|
| Cetearyl lsononanoate | 4,00 | 4,00 | 4,00 |
| Mineral oil | 6,00 | 6,00 | 6,00 |
| Cutina CBS | 2,00 | 2,00 | 2,00 |
| Cutina FS 45 | 1,50 | 1,50 | 1,50 |
| Eumulgin B3 | 1,00 | 1,00 | 1,00 |
| Baysilone-Öl M 350 | 1,00 | 1,00 | 1,00 |
| Tocopherylacetat | 0,50 | 0,50 | 0,50 |
| Propylparaben | 0,30 | 0,30 | 0,30 |
| Pemulen TR 1 | 0,27 | 0,27 | 0,27 |
| Glycerin | 5,00 | 3,00 | 3,00 |
| Milchsäure 80 % | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Panthenol | 0,50 | - | - |
| Seanergilium BG | 1,00 | - | - |
| Natriumchlorid | 0,05 | 0,05 | 0,05 |
| Sepigel 305 | 0,50 | 0,50 | 0,50 |
| Silk Protein | 0,25 | 0,25 | 0,25 |
| Hotact VBE | 0,35 | - | - |
| Herbasol Capsicum Extrakt | - | - | 1,00 |
| Pseudocapsaicin | - | 0,20 | - |
| Calmiskin | 2,00 | - | - |
| N-Palmitoylethanolamin | - | - | 0,50 |
| Ocaline | - | 3,00 | - |
| Calmosensine | 1,00 | 2,00 | - |
| Keltrol SF | 0,20 | 0,20 | 0,20 |
| Hydrovance | 4,30 | 2,50 | 8,60 |
| Natriumascorbylphosphat | 1,00 | - | 1,00 |
| Keratec Pep | - | 2,00 | - |
| Kreatin | - | - | 1,00 |
| Retinol | 0,10 | - | - |
| Deliner | - | 2,00 | - |
| Perfume | 0,30 | 0,30 | 0,30 |
| Aqua | ad 100 | ad 100 | ad 100 |

### 10. Hautcremes auf Basis einer Lipoprotein-Creme

Die Zusammensetzungen 10.1 - 10.6 sind bevorzugte Ausführungsformen von Cremes mit einem Anticellulite-Effekt bzw. einem Effekt zur Verbesserung der Silhouette des Gesichts, insbesondere im Kinn-Bereich, mit erfindungsgemäß verbesserter Hautverträglichkeit und einem Gehalt an diversen Antiageing-Wirkstoffen, auf Basis einer die Hautverträglichkeit besonders unterstützenden Emulsionsbasis mit einem natürlichen Protein-Emulgator (Hibiscin^{®} HP LS 9198).

| | | | | | | |
|---|---|---|---|---|---|---|
| | 10.1 | 10.2 | 10.3 | 10.4 | 10.5 | 10.6 |
| Myritol^{®} PC | 3,5 | 3,5 | 3,5 | 3,5 | 3,5 | 3,5 |
| Lanette^{®} 22 | 3,0 | 3,0 | 3,0 | 3,0 | 3,0 | 3,0 |
| Cutina^{®} GMS-V | 3,0 | 3,0 | 3,0 | 3,0 | 3,0 | 3,0 |
| Stenol^{®} 16/18 | 2,0 | 2,0 | 2,0 | 2,0 | 2,0 | 2,0 |
| Distelöl | 3,0 | 3,0 | 3,0 | 3,0 | 3,0 | 3,0 |
| Controx^{®} KS | 0,05 | 0,05 | 0,05 | 0,05 | 0,05 | 0,05 |
| Baysilon^{®} M350 | 1,0 | 1,0 | 1,0 | 1,0 | 1,0 | 1,0 |
| Isopropylstearat | 6,0 | 6,0 | 6,0 | 6,0 | 6,0 | 6,0 |
| Propylparaben | 0,2 | 0,2 | 0,2 | 0,2 | 0,2 | 0,2 |
| Caomint | 1,00 | - | 2,00 | 1,00 | - | 2,00 |
| Calmosensine | 1,00 | 2,00 | - | 1,00 | 2,00 | - |
| Glycerin | 5,0 | 5,0 | 5,0 | 5,0 | 5,0 | 5,0 |
| Ethoxydiglycol | 5,0 | 5,0 | 5,0 | 5,0 | 5,0 | 5,0 |
| Methylparaben | 0,2 | 0,2 | 0,2 | 0,2 | 0,2 | 0,2 |
| Hibiscin^{®} HP LS 9198 | 3,0 | 3,0 | 3,0 | 3,0 | 3,0 | 3,0 |
| Citronensäure | 0,1 | 0,1 | 0,1 | 0,1 | 0,1 | 0,1 |
| Dihydroxyaceton | 0,5 | - | - | - | - | - |
| Fucoge11000 | 2,0 | 1,0 | 1,0 | 1,0 | - | 1,0 |
| Sepigel^{®} 305 | 2,0 | 2,0 | 2,0 | 2,0 | 2,0 | 2,0 |
| Pearl Protein Extract | - | 2,00 | - | - | 1,00 | - |
| Glistin | 1,00 | - | 2,00 | 2,00 | - | - |
| Kombuchka | 3,00 | - | - | 3,00 | 3,00 | - |
| Natriumascorbylphosphat | - | - | - | - | - | 1,00 |
| Retinol | - | 0,25 | | 0,10 | | 0,15 |
| Deliner | 1,00 | - | 1,00 | - | 1,00 | - |
| Hotact VBE | 0,35 | - | - | 0,2 | - | - |
| Capsicum Extrakt | - | - | 1,00 | - | - | 0,50 |
| Pseudocapsaicin | - | 0,20 | - | - | 0,10 | - |
| Calmiskin | 2,00 | - | - | 2,00 | - | - |
| N-Palmitoylethanolamin | - | - | 0,50 | - | - | 1,00 |
| Ocaline | - | 3,00 | - | - | 3,00 | - |
| Calmosensine | 1,00 | 2,00 | - | 3,00 | 1,50 | - |
| Aqua | ad 100 | ad 100 | ad 100 | ad 100 | ad 100 | ad 100 |

### 11. Reinigungsgele mit Slimming-Effekt bzw. Anticellulite-Effekt:

Die Zusammensetzungen 11.1 - 11.3 sind bevorzugte Ausführungsformen von Reinigungsgelen mit einem Anticellulite-Effekt bzw. einem Effekt zur Verbesserung der Silhouette des Gesichts, insbesondere im Kinn-Bereich, mit erfindungsgemäß verbesserter Hautverträglichkeit.

| | 11.1 | 11.2 | 11.3 |
|---|---|---|---|
| Carbomer | 1,40 | 1,40 | 1,40 |
| Sorbitol | 2,10 | 2,10 | 2,10 |
| Sodium Benzoate | 0,40 | 0,40 | 0,40 |
| Plantaren^{®} 1200 | 7,50 | 7,50 | 7,50 |
| Dehyton^{®} K | 3,40 | 3,40 | 3,40 |
| Texapon^{®} SB 3 | 5,00 | 5,00 | 5,00 |
| Cetiol^{®} HE | 0,50 | 0,50 | 0,50 |
| Lamesoft^{®} PO 65 | 5,00 | 5,00 | 5,00 |
| Controx KS | 0,05 | 0,05 | 0,05 |
| Sodium pyrrolidone carboxylic acid | 1,60 | 1,60 | |
| Pantolactone | 1,00 | 1,00 | |
| Tetrasodium EDTA | 0,25 | 0,25 | 0,25 |
| Sodium Lactate | 1,80 | | |
| Panthenol | 0,50 | 0,50 | 0,50 |
| Caomint | 1,00 | - | 2,00 |
| Calmosensine | 1,00 | 2,00 | - |
| Hotact VBE | 0,35 | - | - |
| Herbasol Capsicum Extrakt | - | - | 1,00 |
| Pseudocapsaicin | - | 0,20 | - |
| Calmiskin | 2,00 | - | - |
| N-Palmitoylethanolamin | - | - | 0,50 |
| Ocaline | - | 3,00 | - |
| Perfume | 0,40 | 0,40 | 0,40 |
| Aqua | ad 100 | ad 100 | ad 100 |

### 12. Zweiphasen-Anticellulite-Massagefluid für die Körperhaut

Die Zusammensetzungen 12.1 und 12.2 sind bevorzugte Ausführungsformen von Massagefluiden mit einem Anticellulite-Effekt bzw. einem Effekt zur Verbesserung der Silhouette des Gesichts, insbesondere im Kinn-Bereich, mit erfindungsgemäß verbesserter Hautverträglichkeit, auf Basis einer Zweiphasenzusammensetzung, die direkt vor dem Gebrauch durch Schütteln in eine Emulsion überführt wird und sich besonders leicht in die Haut einmassieren lässt. Die Zusammensetzung ruft ein angenehm erfrischendes Hautgefühl hervor.

| | 12.1 | 12.2 |
|---|---|---|
| Allantoin | 0,100000 | 0,100000 |
| D&C Green No. 5 | 0,000800 | 0,000800 |
| Milchsäure 80% DAB | 0,100000 | 0,100000 |
| Dinatriumphosphat wasserfrei | 0,100000 | 0,100000 |
| D-Panthenol 75 % | 0,100000 | 0,100000 |
| Parfum | 0,150000 | 0,150000 |
| Brij 30 | 0,500000 | 0,500000 |
| Trisiloxane Fluid 1 cs | 20,000000 | 20,000000 |
| Hotact VBE | 0,35 | - |
| Herbasol Capsicum Extrakt | - | 2,00 |
| Caomint | 2,000000 | - |
| Calmosensine | - | 2,000000 |
| Ethanol 96 % | 25,000000 | 25,000000 |
| Wasser, vollentsalzt | ad 100,000000 | ad 100,000000 |

### 13. Beispiele für Zusammensetzungen zum Tränken von Tüchern

| | Bestandteile | 13.1 | 13.2 | 13.3 | 13.4 |
|---|---|---|---|---|---|
| Phase 1 | PEG-40 Hydrogenated Castor Oil | 0,1 | 0,1 | 0,1 | - |
| | Trideceth-9 | 0,1 | 0,1 | 0,1 | - |
| | Parfum | 0,3 | 0,1 | 0,1 | 0,1 |
| Phase 2 | Hexyllaurat | 2,0 | - | - | - |
| | Dicaprylylcarbonat | - | - | 2,0 | - |
| | Dipropylenglycol | - | 2,0 | - | - |
| Phase 3 | Panthenol | 0,5 | - | - | - |
| | Citronensäure (Monohydrat) | 1,5 | 1,5 | 1,5 | 1,5 |
| | Trinatriumcitrat (Dihydrat) | 2,0 | 2,0 | 2,0 | 2,0 |
| | Natriumsalicylat | 0,45 | - | - | - |
| | Decylglucosid | - | - | - | 2,0 |
| | Pemulen TR 1 | - | - | - | 0,2 |
| | Hotact VBE | 0,35 | - | 0,10 | - |
| | Herbasol Capsicum Extrakt | - | 0,5 | - | 1,0 |
| | Caomint | - | 3,00 | 3,00 | - |
| | Calmosensine | 3,00 | - | - | 2,00 |
| | Wasser | ad 100 | ad 100 | ad 100 | ad 100 |

Mit diesen Tränkzusammensetzungen wurden verschiedene Viskose-Vliesstoffe ausgerüstet. Es wurden gelochte, ungelochte, genoppte, einlagige, zweilagige und dreilagige Vliese ausgerüstet. Die Tücher wurden sowohl als feuchte Tücher als auch als trockene Tücher (das heißt, nach dem Ausrüsten wurden die Tücher bis auf einen Restwassergehalt kleiner 10 Gew.%, bevorzugt kleiner 5 Gew.-%, getrocknet, um direkt vor Gebrauch mit einer kleinen Menge an Wasser wieder befeuchtet zu werden oder auf feuchter Haut angewendet zu werden) verwendet.
Als besonders bevorzugt erwiesen sich genoppte Tücher (beispielsweise mit heiß aufgesprühten Kunststoffnoppen) und Tücher mit grober Oberflächenstruktur, da diese den gewünschten Massageeffekt besonders vorteilhaft unterstützen.
Derartige Slimming-Tücher sind besonders vorteilhaft zur Anwendung unterwegs.

### 14. Matrixpflaster mit Anti-Cellulite-Effekt

| | 14.1 | 14.2 | 14.3 | 14.4 | 14.5 |
|---|---|---|---|---|---|
| DURO-TAK^{®} 387-2516 | 76 | 76 | 76 | 76 | 76 |
| Natriumascorbylphosphat | 3 | 2 | 3 | - | 2 |
| Magnesiumascorbylphosphat | - | - | - | 2 | 1 |
| Vitamin B6 | 0,05 | 0,05 | - | 0,05 | - |
| Naringin | 0,1 | - | - | - | - |
| Calmosensine | 0,5 | - | - | 0,5 | - |
| Caomint | 1,0 | - | - | 2,0 | 2,0 |
| Enteline 2 | - | 3,0 | 2 | - | - |
| Aloe Vera Gel | 1 | - | 1 | - | - |
| Propylenglycolmonooleat | 5 | 5 | - | - | - |
| Tween^{®}-80 | - | - | 5 | 3 | 5 |
| Natriumcitrat | 0,1 | 0,1 | 0,1 | 0,1 | 0,1 |
| Controk^{®} KS | 0,05 | 0,05 | 0,05 | 0,05 | 0,05 |
| Herbasol Extrakt Capsicum | - | - | - | 1,0 | - |
| Nonivamid | - | - | - | - | 0,5 |
| Benzylnicotinat | 0,5 | - | - | - | - |
| Ethylnicotinat | - | - | 0,5 | - | - |
| Nicoboxil | - | 0,5 | - | - | - |
| Wasser | ad 100 | ad 100 | ad 100 | ad 100 | ad 100 |

### 15. Reservoirpflaster mit Anti-Cellulite-Effekt

| Bestandteile des Wirkstoffreservoirs | 15.1 | 15.2 |
|---|---|---|
| Enteline 2 | - | 2,0 |
| Ocaline | 2,0 | - |
| Herbasol Extrakt Capsicum | 1,0 | - |
| Ethylnicotinat | - | 0,2 |
| Ederline H | 1,0 | 1,0 |
| Carbopol^{®} 980 | 0,5 | - |
| Pemulen^{®} TR 1 | - | 0,5 |
| NaOH | 0,1 | 0,1 |
| Titandioxid | 0,2 | 0,2 |
| Ethanol | 1,0 | 1,0 |
| Polyvinylalkohol | 2,0 | 1,0 |
| Carboxymethylcellulose | 1,0 | 0,5 |
| Glycerin | 10 | 20 |
| Sorbitol | 7,0 | 5,0 |
| 1,3-Butandiol | 3,0 | 3,0 |
| Tween^{®}-80 | 0,05 | 0,05 |
| Paraffinöl | 5,0 | 2,0 |
| Natriumcitrat | 0,1 | 0,1 |
| Controx KS | 0,05 | 0,05 |
| Wasser | ad 100 | ad 100 |

Die Bestandteile der Wirkstoffreservoir-Rezepturen 15.1 bzw. 15.2 wurden miteinander vermischt, so dass eine gelartige Masse entstand. Mit dieser Masse wurden Pflasterträger beschichtet, so dass sogenannte Reservoirpflaster erhalten wurden. Diese Pflaster werden sowohl auf die trockene Haut als auch auf die angefeuchtete Haut gelegt und verbleiben dort für eine Zeit von wenigen Sekunden bis einigen Stunden.

### 16. Körpercreme

Die nachfolgende Rezeptur 16.1 ist ein bevorzugtes Beispiel für eine erfindungsgemäße Anticellulite-Körpercreme.

| | |
|---|---|
| Paraffinum Liquidum | 20,00 |
| Arlacel 165 | 4,00 |
| Eutanol G | 1,00 |
| Cetiol SN | 1,50 |
| Cremophor A 6 | 2,00 |
| Behenylalkohol | 1,60 |
| Silikonöl 350 cs | 3,00 |
| Brij 721 VP | 0,40 |
| Propylparaben | 0,20 |
| Controx KS | 0,25 |
| Propandiol-1,2 | 3,00 |
| Glycerin 86% pflanzlich | 5,00 |
| Polyethylenglykol MG 400 | 1,70 |
| Methylparaben | 0,20 |
| Phenoxyethanol, rein | 0,50 |
| Carbopol ETD 2020 | 0,50 |
| Natriumhydroxid Perlen | 0,03 |
| Citronensäure Monohydrat | 0,10 |
| Trinatriumcitrat Dihydrat | 0,10 |
| Herbasol Extrakt Capsicum | 3,00 |
| Enteline 2 | 2,00 |
| Parfum | 0,30 |
| Simulgel NS | 1,00 |
| Wasser | ad 100,00 |

### Liste der verwendeten Rohstoffe

| Handelsname | INCI-Bezeichnung | Lieferant/Hersteller |
|---|---|---|
| | | |
| Abil EM 90 | CETYL PEG/PPG-10/1 DIMETHICONE | Degussa |
| Arlacel 165 | Glyceryl Stearate, PEG-100 Stearate | Uniqema |
| Baysilone-Öl M 350 | Dimethicone | GE Bayer Silicones |
| Belsil DM 100 | Dimethicone (100 cSt) | Wacker |
| Brij 30 | Laureth-4 | Uniqema |
| Brij 721 VP | STEARETH-21 | Uniqema |
| Calmiskin | Water (and) Mentha piperita (Peppermint) Leaf Extract | Silab |
| Calmosensine | Butylene Glycol, Water, Laureth-3, Hydroxy-ethylcellulose, Acetyl Dipeptide-1 Cetylester | Sederma |
| Caomint | Propylene Glycol, Aqua, Mentha piperita, Theobroma cacao | Solabia |
| Carbopol 980 | Carbomer | Noveon |
| Carbopol ETD 2020 Cetiol B | Acrylates/C10-30 Alkyl Acrylate Crosspolymer | Noveon |
| | Dibutyl Adipate | Cognis |
| Cetiol^{®} HE | PEG-7 Glyceryl Cocoate | Cognis |
| Cetiol^{®} SB 45 | Butyrospermum parkii (Shea butter) | Cognis |
| Cetiol^{®} SN | Cetearyl Isononanoate | Cognis |
| Controx KS | Tocopherol, Hydrogenated Palm Glycerides Citrate | Cognis |
| Cremophor A 6 | CETEARETH-6, STEARYL ALCOHOL | BASF |
| Cutina CBS | Glyceryl Stearate / Cetearyl Alcohol / Cetyl Palmitate / Cocoglycerides im Gewichtsverhältnis 7:1:1:1 | Cognis |
| Cutina GMS-V | Glyceryl Stearate (Mischung aus Glycerylmono-und distearat) | Cognis |
| Cutina MD | Glyceryl Stearate (Mischung aus Glycerylmono-und distearat) | Cognis |
| Dehyton K | AQUA (WATER), COCAMIDOPROPYL BETAINE (29 - 32 Gew.-%) | Cognis |
| Deliner | Zea Mays (Corn) Kernel Extract, Butylene Glycol, Xanthan Gum | Coletica |
| Dow Corning 1501 Fluid | Cyclomethicone (85 - 86 Gew.-%), Dimethiconol (14-15 Gew.-%) | Dow Corning |
| Dow Corning 200 | Dimethicone (0,65 cSt) | Dow Corning |
| Dow Corning^{®}200 Fluid, | Dimethicone (5 cSt) | Dow Corning |
| Dow Corning^{®}245 Fluid | Cyclomethicone | Dow Corning |
| Dow Corning 9040 | Cyclomethicone/ Dimethicone Crosspolymer | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinylpyrrolidon | National Starch and Chemical |
| EDENOR L2 S M | Palmitic Acid, Stearic Acid (ca. 1: 1) | Cognis |
| Ederline H | PEG-40 Hydrogenated Castor Oil, PPG-2-Ceteareth-9, Pyrus Malus (Apple) Fruit Extract | Seporga |
| Elfacos ST 37 | PEG-45/Dodecyl Glycol Copolymer | Akzo Nobel |
| Enteline 2 | Butylene Glycol, Glycerine, Hydrolyzed Enteromorpha compressa | BiotechMarine |
| Eumulgin B 3 | Ceteareth-30 | Cognis |
| Eutanol G | OCTYLDODECANOL | Cognis |
| Fucogel 1000 | Biosaccharide Gum-1 | Solabia |
| Glistin | Wasser, L-Glutamylaminoethyl indole | Exsymol |
| Herbasol Extrakt Capsicum | Propylene Glycol, Aqua (Water), Capsicum Frutescens Fruit Extract, Sorbitol | Cosmetochem |
| Hibiscin^{®} HP-LS-9198 | Water, Hibiscus esculentus Seed Extract, Phenoxyethanol | Laboratoires Sérobiologiques |
| Hotact VBE | Vanillyl Butyl Ether | Takasago |
| Hydagen CMF | Chitosan Glycolate | Cognis |
| Hydrovance | Wasser, Bis-N,N'-(2-Hydroxyethyl)harnstoff, Harnstoff, Ammoniumlactat | National Starch |
| Keltrol SF | Xanthan Gum | Kelco |
| Keratec Pep | Water, Hydrolyzed Keratin | Croda |
| Keratolite | Aqua (Water), Malic Acid, Butylene Glycol, Prunus Amygdalus Dulcis (Sweet Almond) Fruit Extract, Sodium Benzoate, Phenoxy-ethanol, Methylparaben, Propylparaben, Ethylparaben | Coletica |
| Kombuchka | Saccharomyces/Xylinum/Black Tea Ferment, Glycerin, Hydroxyethylcellulose | Sederma |
| Lameform TGI | Polyglyceryl-3 Diisostearate | Cognis |
| Lamesoft^{®} PO 65 | Coco-Glucoside, Glyceryl Oleate, Water | Cognis |
| LANETTE^{®} 22 | Behenyl Alcohol | Cognis |
| Lipochroman 6 | DIMETHYLMETHOXY CHROMANOL | Lipotec |
| Lipoid S 75-3 | Aqua, Lecithine | Lipoid GmbH |
| Lotus Germ Extract | Water, Butylene Glycol, Nelumbo Nucifera Germ Extract | Maruzen |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Montanov L | C14-22 Alcohols/C12-20 Alkyl Glucoside | Seppic |
| Myritol 318 | Caprylic/Capric Triglyceride | Cognis |
| Myritol^{®} PC | PROPYLENE GLYCOL DICAPRYLATE/DICAPRATE | Cognis |
| Natipide II PG | Aqua, Propylene Glycol, Lecithin | Phospholipid GmbH |
| Natrulon RC 50 DG | WATER, CARNITINE, POLYGLYCERIN-10 | Lonza |
| Neo Heliopan AP | Disodium Phenyl Dibenzimidazole Tetrasulfonate | Symrise |
| Novata AB | Cocoglycerides | Cognis |
| Ocaline | Sea Water (and) Water (and) Cucurbita pepo (pumpkin) seed extract | Soliance |
| Olea europ. Fol extr. S. sicc. | Olea Europea (Olive) Leaf Extract | Fruitarom |
| Parsol 1789 | BUTYL METHOXYDIBENZOYLMETHANE | DSM |
| Parsol 340 | Octocrylene | DSM |
| Parsol HS | PHENYLBENZIMIDAZOLE SULFONIC ACID | DSM |
| Parsol SLX | Polysilicone-15 | DSM |
| Pearl Protein Extract | Aqua, Hydrolyzed Conchiolin Protein | Maruzen |
| Pemulen TR 1 | Acrylates/C10-30 Alkyl Acrylate Crosspolymer | Noveon |
| Photosomes | Aqua, Lecithine, Plancton Extract | AGI Dermatics |
| Phytokine | Aqua, Butylene Glycol, HYDROLYZED SOY PROTEIN, Methylparaben, Ethylparaben, Propylparaben | Coletica |
| Plantaren^{®} 1200 | Lauryl Glucoside, ca. 50 % Aktivsubstanz | Cognis |
| Ridulisse C | HYDROLYZED SOY PROTEIN | Silab |
| Sambucus AO | Water Glycerin, Sambucus Nigra Flower Extract | Alpaflor/Centerchem |
| Seanergilium BG | Butylene Glicol, Laminaria Digitata Extract, Methylparaben | Coletica |
| Sepigel^{®}305 | Aqua (Water) Polyacrylamide, C₁₃-C₁₄ Isopar-affin, Laureth-7 (Aktivsubstanz Verdicker-polymer ca. 45 - 49 Gew.-%) | SEPPIC |
| Simulgel NS | Aqua (Water)/Hydroxyethyl Acrylate /Sodium Acryloyldimethyl Taurate Copolymer /Squalane / Polysorbate 60 | Seppic |
| Synperonic PE L 64 | Poloxamer-184 | Uniqema |
| Softisan 649 | Bis-Diglyceryl Polyacyladipate-2 | Sasol |
| Stenol 16/18 | Cetearylalkohol | Cognis |
| Symdiol 68 | 1,2-Octandiol, 1,2-Hexandiol | Symrise |
| Tego Carbomer 140 | Carbomer | Degussa |
| Texapon^{®} SB 3 | Aqua, Disodium Laureth Sulfosuccinate, (ca. 40 % Aktivsubstanz), Citric Acid | Cognis |
| Tween^{®}-80 | Polysorbate-80 | |
| Uvinul MBC 95 | 4-METHYLBENZYLIDENE CAMPHOR | BASF |

## Patentansprüche

1. Kosmetische oder dermatologische Zusammensetzung zur topischen Behandlung der Haut, enthaltend in einem geeigneten kosmetischen oder dermatologischen Träger
a) mindestens einen hyperämisierenden Wirkstoff und
b) mindestens einen Wirkstoff, der die Degranulierung von Mastzellen und/oder die Ausschüttung von Histamin reduziert.

2. Kosmetische oder dermatologische Zusammensetzung gemäß Anspruch 1, **dadurch gekennzeichnet, dass** der hyperämisierende Wirkstoff a) ausgewählt ist aus
- Nicotinsäureestern, insbesondere Nicotinsäurebenzylester (Benzylnicotinat), Nicotinsäurebutoxyethylester (Nicoboxil), Nicotinsäuremethylester, Nicotinsäureethylester, Nicotinsäurehexylester, Nicotinsäureisopropylester, Nicotinsäuremyristylester, Tetrahydrofurfuryl-Nicotinat (Thurfyl-Nicotinat) und Tetrahydrofurfuryl-Nicotinat-Hydrochlorid,
- Pyridin-3-carbaldehyd (β-Pyridincarbaldehyd, Nicotinaldehyd),
- Salicylsäureestern, insbesondere Phenylsalicylat,
- Vanillylethern, insbesondere Vanillylbutylether,
- Carbonsäurevanillylamiden, insbesondere Nonivamid,
- Scharfstoffen, insbesondere Capsaicin, Cantharidin, Piperin, Gingerol, Zingeron, Myristicin, Safrol, Allicin, Sedamin, Sacculatal, Chalciporon, Isochalciporon, Velleral, Vellerol, und Isothiocyanaten (Senfölen), insbesondere Benzylsenföl,
- Extrakten aus Scharfstoff-Pflanzen, insbesondere aus Capsicum, insbesondere aus Capsicum annuum und Capsicum frutescens, aber auch aus Capsicum chinense, Capsicum baccatum und Capsicum pubescens, weiterhin aus Mauerpfeffer und aus der Zaunrübe,
- Terpentinöl,
- sowie Mischungen hiervon.

3. Kosmetische oder dermatologische Zusammensetzung gemäß einem der vorstehenden Ansprüche, **dadurch gekennzeichnet, dass** der Wirkstoff b), der die Degranulierung von Mastzellen und/oder die Ausschüttung von Histamin reduziert, ausgewählt ist aus
- Extrakten aus den Samen von Cucurbita pepo (Zucchini),
- Extrakten aus Mentha piperita, insbesondere aus den Blättern von Mentha piperita,
- N-acylierten Alkanolaminen, insbesondere N-Palmitoylethanolamin,
- Hydrolysaten oder Extrakten aus der Grünalge *Enteromorpha compressa,*
- dem Dipeptid Tyr-Arg, dessen Estern und dessen N-Acyl-Derivaten, insbesondere N-Acetyl-Tyr-Arg-hexyldecylester,
- sowie Mischungen hiervon.

4. Kosmetische oder dermatologische Zusammensetzung gemäß einem der vorstehenden Ansprüche, enthaltend mindestens einen weiteren Wirkstoff, der die Kollagensynthese stimuliert.

5. Kosmetische oder dermatologische Zusammensetzung gemäß einem der vorstehenden Ansprüche, enthaltend mindestens einen weiteren Wirkstoff, der die Interaktion zwischen der extrazellulären Matrix und den Fibroblasten erhöht und/oder verbessert.

6. Kosmetische oder dermatologische Zusammensetzung gemäß einem der vorstehenden Ansprüche, enthaltend mindestens einen weiteren kosmetischen Wirkstoff, ausgewählt aus:
a) Monomeren und Oligomeren von Aminosäuren, N-C₂-C₂₄-Acylaminosäuren, den Estern und/oder den physiologisch verträglichen Metallsalzen dieser Substanzen, die nicht die Degranulierung von Mastzellen und/oder die Ausschüttung von Histamin reduzieren können,
b) DNA-Reparaturenzymen,
c) DNA- oder RNA-Oligonucleotiden,
d) Vitaminen, Provitaminen und Vitaminvorstufen der Gruppen B, E, H und K und den Estern der vorgenannten Substanzen,
e) α-Hydroxycarbonsäuren, α-Ketocarbonsäuren, β-Hydroxycarbonsäuren und deren Ester-, Lacton- und/oder Salzform,
f) Flavonoiden und Flavonoid-reichen Pflanzenextrakten, die nicht die Degranulierung von Mastzellen und/oder die Ausschüttung von Histamin reduzieren können,
g) Ubichinon und Ubichinol sowie deren Derivaten,
h) Silymarin,
i) Ectoin,
j) anorganischen und organischen UV-Filtersubstanzen,
k) selbstbräunenden Wirkstoffen,
l) hautberuhigenden Wirkstoffen,
m) feuchtigkeitsspendenden Wirkstoffen,
n) sebumregulierenden Wirkstoffen,
o) sowie Mischungen dieser Wirkstoffe a) - n).

7. Kosmetische oder dermatologische Zusammensetzung gemäß einem der vorstehenden Ansprüche, **dadurch gekennzeichnet, dass** die Monomeren von Aminosäuren, N-C₂-C₂₄-Acylaminosäuren und/ oder den physiologisch verträglichen Metallsalzen dieser Substanzen, die nicht die Degranulierung von Mastzellen und/oder die Ausschüttung von Histamin reduzieren können, ausgewählt sind aus
- Alanin, Arginin, Asparagin, Asparaginsäure, Canavanin, Citrullin, Cystein, Cystin, Desmosin, Glutamin, Glutaminsäure, Glycin, Histidin, Homophenylalanin, Hydroxylysin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat,
- sowie Mischungen dieser Substanzen.

8. Kosmetische oder dermatologische Zusammensetzung gemäß einem der vorstehenden Ansprüche, **dadurch gekennzeichnet, dass** mindestens ein nichtionischer Emulgator mit einem HLB-Wert von 8 und darunter der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen darstellt, enthalten ist.

9. Kosmetische oder dermatologische Zusammensetzung gemäß einem der vorstehenden Ansprüche, **dadurch gekennzeichnet, dass** sie in Form einer lamellare Strukturen aufweisenden Öl-in-Wasser-Emulsion vorliegt.

10. Kosmetische oder dermatologische Zusammensetzung gemäß einem der vorstehenden Ansprüche, **dadurch gekennzeichnet, dass** sie in Form einer Wasser-in-C51-Emulsion vorliegt.

11. Kosmetische oder dermatologische Zusammensetzung gemäß einem der vorstehenden Ansprüche, **dadurch gekennzeichnet, dass** sie in einem Sprühspender oder Pumpspender verpackt ist.

12. Nicht-therapeutische, kosmetische Verwendung einer kosmetischen oder dermatologischen Zusammensetzung gemäß einem der Ansprüche 1 - 11 zur Behandlung der Cellulite.

13. Nicht-therapeutische kosmetische Verwendung einer kosmetischen oder dermatologischen Zusammensetzung gemäß einem der Ansprüche 1 - 11 zur Steigerung der Hautfestigkeit und/oder zur Verbesserung der mechanischen Stabilität der Haut und/oder zur Steigerung der Hautelastizität und/oder zur Straffung der Haut.

14. Nicht-therapeutische kosmetische Verwendung einer kosmetischen oder dermatologischen Zusammensetzung gemäß einem der Ansprüche 1 - 11 zur Glättung der Haut und/oder zur Verminderung von Falten, Fältchen und/oder feinen Linien und/oder zur Verbesserung des optischen Erscheinungsbildes der Haut.

15. Verwendung einer Wirkstoffkombination aus
a) mindestens einem hyperämisierenden Wirkstoff und
b) mindestens einem Wirkstoff, der die Degranulierung von Mastzellen und/oder die Ausschüttung von Histamin reduziert,
zur Herstellung einer kosmetischen oder dermatologischen Zusammensetzung
- zur Behandlung der Cellulite und/oder
- zur Steigerung der Hautfestigkeit und/oder
- zur Verbesserung der mechanischen Stabilität der Haut und/oder
- zur Steigerung der Hautelastizität und/oder
- zur Straffung der Haut und/oder
- zur Glättung der Haut und/oder
- zur Verminderung von Falten, Fältchen und/oder feinen Linien und/oder
- zur Verbesserung des optischen Erscheinungsbildes der Haut.

16. Verwendung mindestens eines Wirkstoffs, der die Degranulierung von Mastzellen und/oder die Ausschüttung von Histamin reduziert, zur Verbesserung der Hautverträglichkeit von Hautbehandlungsmitteln mit mindestens einem hyperämisierenden Wirkstoff, insbesondere von Anticellulitemitteln.

17. Verwendung gemäß Anspruch 16, **dadurch gekennzeichnet, dass** der mindestens eine Wirkstoff, der die Degranulierung von Mastzellen und/oder die Ausschüttung von Histamin reduziert, ausgewählt ist aus
- Extrakten aus den Samen von Cucurbita pepo (Zucchini),
- Extrakten aus Mentha piperita, insbesondere aus den Blättern von Mentha piperita,
- N-acylierten Alkanolaminen, insbesondere N-Palmitoylethanolamin,
- Hydrolysaten oder Extrakten aus der Grünalge *Enteromorpha compressa,*
- dem Dipeptid Tyr-Arg, dessen Estern und dessen N-Acyl-Derivaten, insbesondere N-Acetyl-Tyr-Arg-hexyldecylester,
- sowie Mischungen hiervon.
